# FASCICULE DE BREVET EUROPEEN

(11) **EP 3 302 421 B1**
(45) Date de publication et mention de la délivrance du brevet: **07.07.2021**
(21) Numéro de dépôt: 16731621.5
(22) Date de dépôt: 27.05.2016
(51) Int. Cl.: A61K 9/10, A61K 9/50, A61P 17/00, A61K 31/402, A61K 31/451, A61K 31/496

(54) **COMPOSITIONS COMPRENANT AU MOINS UN PRINCIPE ACTIF DISPERSE ET DES MICROCAPSULES LIPIDIQUES**
ZUSAMMENSETZUNGEN MIT MINDESTENS EINEM DISPERGIERTEN WIRKSTOFF UND LIPID-MIKROKAPSELN
COMPOSITIONS COMPRISING AT LEAST ONE DISPERSED ACTIVE PRINCIPLE AND LIPID MICROCAPSULES

(30) Priorité: 29.05.2015 US 201562168085 P
(43) Date de publication de la demande: 11.04.2018
(73) Titulaire: Galderma Research & Development, 06410 Biot (FR)
(72) Inventeur: DUBAYLE, Carole, 06370 Mouans Sartoux (FR); MALLARD, Claire, 06250 Mougins (FR); ATRUX-TALLAU, Nicolas, 06800 Antibes (FR)
(74) Mandataire: Nederlandsch Octrooibureau
(86) Numéro de dépôt international: PCT/FR2016/051261
(87) Numéro de publication internationale: WO 2016/193588

(56) Documents cités:
- WO-A1-95/03829
- WO-A1-2010/063774
- WO-A1-2015/082659
- WO-A2-2010/072958
- WO-A2-2010/133609
- WO-A2-2014/140861
- US-A- 4 902 513

## Description

### DOMAINE DE L'INVENTION

La présente invention concerne une composition pharmaceutique. L'invention concerne aussi l'utilisation de la composition pour améliorer la libération et pénétration de l'actif, et son utilisation pour le traitement de diverses pathologies liées à l'actif utilisé.

### ARRIERE PLAN DE L'INVENTION

L'homme de l'art est en permanence confronté à des difficultés de mise en formulation des actifs, notamment pharmaceutiques, d'une part à cause de leur solubilité au sein de la composition qui peut s'avérer défavorable, et d'autre part à cause de leur stabilité.

Pour atteindre le niveau de solubilisation souhaité, il est parfois nécessaire d'incorporer une forte concentration de solubilisant, en général des excipients ou des solvants, difficiles à mettre en œuvre en formulation et particulièrement mal tolérés à forte concentration selon les pathologies traitées. Par ailleurs, une fois solubilisé, l'actif se doit d'être stable ainsi que la composition le contenant.

Or, il est connu que les actifs incorporés sous forme solubilisée sont en général moins stables dans une composition que ces mêmes actifs incorporés sous forme dispersée au sein de la composition.

De même, les actifs incorporés sous forme solubilisée sont souvent plus irritants que ces mêmes actifs incorporés sous forme dispersée au sein de la composition. Par contre, les actifs incorporés sous forme dispersée présentent plus de difficultés pour pénétrer dans la peau comparés à ces mêmes actifs sous forme solubilisée au sein de la composition.

Ainsi, il persiste un besoin de formuler des actifs dans des compositions stables avec une pénétration améliorée de ces actifs dans la peau.

### RESUME DE L'INVENTION

Le problème que se propose de résoudre ici la présente invention, est donc de concevoir une composition comprenant au moins un principe actif dispersé dans la composition, la dite composition devant être stable physiquement et chimiquement et capable d'assurer une bonne pénétration dans la peau du principe actif dispersé, ainsi qu'une bonne tolérance dans la pathologie ciblée.

La demanderesse a découvert de manière surprenante que la présence de microcapsules lipidiques dispersées au sein d'un véhicule d'une composition permet d'aider l'actif dispersé au sein du même véhicule à pénétrer dans les couches cutanées après application sur la peau. La demanderesse a ainsi démontrée que, malgré la présence du principe actif sous forme dispersée au sein de la composition, celui-ci peut pénétrer dans la peau autant que si le principe actif était sous forme solubilisée. La demanderesse a, de plus découvert, de façon particulièrement surprenante, qu'il n'était pas forcément nécessaire que le contenu du cœur huileux des microcapsules soit très solvant du principe actif pour obtenir une bonne pénétration de l'actif dispersé.

La présente invention concerne donc une composition pharmaceutique comprenant au moins un principe actif dispersé au sein d'un véhicule pharmaceutiquement acceptable, et des microcapsules de taille micrométrique telles que décrites dans la revendication 1.

Dans la composition de l'invention, et à la différence de l'art antérieur, le principe actif n'est pas présent au sein des microcapsules lipidiques et se trouve au sein d'un véhicule pharmaceutiquement acceptable. La composition de l'invention a notamment pour objectif de garantir la stabilité du principe actif en l'intégrant dans la composition sous forme dispersée et plus particulièrement d'améliorer la pénétration dans la peau de ce principe actif malgré son état dispersé. La composition de l'invention est bien tolérée avec une texture sensorielle intéressante adaptée à la pathologie à traiter.

Dans un mode de réalisation préféré, le principe actif dispersé est le Trifarotène, le (S)-4-(2,4-dihydroxyphenyl)-N-(1-phenylethyl)piperidine-1-carboxamide, ou le (S)-N-hydroxy-3-[4-(2-méthyl-quinolin-4-ylméthoxy)-benzènesulfonylamino]-2-[4-(propane-2-sulfonyl)-pipérazin-1-yl]-propionamide.

Les microcapsules de taille micrométrique contiennent une phase interne huileuse, et une enveloppe non polymérique obtenue à partir d'au moins un composé lipidique choisi parmi les lipides amphiphiles.

De préférence, le composé lipidique constituant l'enveloppe des microcapsules est choisi parmi les lécithines hydrogénée ayant une quantité en poids de phosphatidylcholine supérieure à 85%. De préférence, le composé lipidique constituant l'enveloppe des microcapsules est présent en une quantité comprise entre 0,01 et 10%, de préférence entre 0,05 et 5% en poids, et plus préférentiellement entre 0,1 et 1% en poids par rapport au poids total de la composition.

De préférence, la phase interne huileuse des microcapsules comprend au moins un corps gras liquide ou semi-liquide à température ambiante (15-25 °C à pression atmosphérique). Plus préférentiellement, la phase interne huileuse est composée de triglycérides, d'esters d'acide gras, d'éthers de polyéthylène glycol, ou du diméthyl isosorbide.

Dans un mode préféré, les microcapsules sont exemptes de co-surfactant, de solvant organique volatil ou de polymère.

Les microcapsules lipidiques de taille micrométrique sont dispersées dans une phase aqueuse. Plus particulièrement, le profil de distribution des microcapsules est tel que 50 % des microcapsules présentent au moins une taille moyenne comprise entre 1 et 80 µm, préférentiellement comprise entre 1 et 50 µm, et plus particulièrement comprise entre 1 et 20 µm.

Un objet préféré de l'invention concerne une composition comprenant 0,001 à 1 % en poids d'un actif dispersé, de préférence le Trifarotène, le (S)-4-(2,4-dihydroxyphenyl)-N-(1-phenylethyl)piperidine-1-carboxamide, ou le (S)-N-hydroxy-3-[4-(2-méthyl-quinolin-4-ylméthoxy)-benzènesulfonylamino] -2- [4- (propane-2- sulfonyl)-pipérazin-1-yl] - propionamide, au sein d'un véhicule pharmaceutiquement acceptable, par rapport au poids total de la composition, et des microcapsules lipidiques dispersées au sein du même véhicule composées de :
a. 0,1 à 5% de lécithine hydrogénée avec une teneur en phosphatidylcholine hydrogénée supérieure à 85% ; et
b. 1 à 30% de corps gras huileux et éventuellement non huileux, de préférence des triglycérides, des esters d'acide gras, des éthers de polyéthylène glycol, et du diméthyl isosorbide.

Dans un mode de réalisation particulier, le véhicule pharmaceutiquement acceptable de la composition de l'invention est un gel.

Dans un autre mode de réalisation particulier, le véhicule pharmaceutiquement acceptable de la composition de l'invention est une crème.

De préférence, la composition de l'invention se présente sous une forme adaptée pour une administration topique.

Un autre objet de l'invention concerne l'utilisation d'une composition telle que définie dans la présente invention pour améliorer la pénétration dans la peau d'un principe actif dispersé.

Un autre objet de l'invention concerne également l'utilisation de microcapsules lipidiques telles que définies dans la présente invention pour améliorer la pénétration dans la peau d'un principe actif dispersé.

L'invention concerne également une composition telle que définie dans la présente demande pour son utilisation en tant que médicament.

L'invention concerne aussi une composition telle que définie dans la présente demande pour son utilisation dans le traitement des affections dermatologiques. De préférence, les affections dermatologiques sont l'acné, les ichtyoses, les états ichtyosiformes, l'hyperkératose palmoplantaire ou le psoriasis.

Un objet supplémentaire de l'invention concerne un procédé de préparation d'une composition telle que définie dans la présente demande comprenant les étapes suivantes :
(i) préparation de l'émulsion primaire par :
   a) préparation d'une phase huileuse chauffée à 75°C,
   b) dispersion du composé lipidique, et plus préférentiellement de la lécithine hydrogénée, dans une phase aqueuse, chauffée à 75°C,
   c) incorporation de la phase huileuse sur la phase aqueuse déjà présente dans l'appareil sous agitation à une vitesse inférieure à 16 000 tr/min, et
   d) laisser circuler le mélange jusqu'au retour à température ambiante ; et
(ii) incorporation de l'émulsion primaire dans le véhicule pharmaceutiquement acceptable contenant le principe actif dispersé.

### LEGENDE DES FIGURES

**Figure 1** **:** Evaluation de la quantité pénétrée (% dose appliquée) dans différents compartiments de la peau des Gels IV/G-A11 et VI/G-A11 de l'exemple 3.
**Figure 2** **:** Profil de pénétration des Gels IV/G-A11 et VI/G-A11 de l'exemple 3 sur 24 heures.
**Figure 3** **:** Représentation des ASC (aires sous la courbe) après 8 heures des Gels IV/G-A11 et VI/G-A11 de l'exemple 3.
**Figure 4** : Représentation des ASC (aires sous la courbe) après 24 heures des Gels IV/G-A11 et VI/G-A11 de l'exemple 3.
**Figure 5** **:** Evaluation de la quantité pénétrée (% dose appliquée) dans différents compartiments de la peau des Gels V/G-A21 et VIII/G-A21 de l'exemple 8.

### DESCRIPTION DETAILLEE DE L'INVENTION

De nombreux actifs, notamment pharmaceutiques, présentent souvent des difficultés de solubilisation, limitant donc leur incorporation dans les véhicules classiquement utilisés, et rendant difficile l'obtention de composition stable et efficace. Par ailleurs, l'incorporation du principe actif sous une forme dispersée influe souvent négativement sur la pénétration du principe actif dans la peau, et ne présente pas une solution idéale au problème rencontré.

La demanderesse a ainsi découvert que la composition selon la présente invention résolvait les problèmes énoncés précédemment. La composition selon l'invention contient donc des microcapsules, au moins un principe actif, tous les deux dispersés au sein d'un même véhicule pharmaceutiquement acceptable, le principe actif étant connu de l'homme de l'art comme présentant des difficultés de solubilisation, de stabilité, de pénétration et/ou de tolérance sous forme solubilisée.

A titre d'exemples non limitatifs de principes actifs compris dans les compositions de l'invention, on peut citer notamment :
- les actifs difficilement solubilisés et stables dans tout type de milieu et en particulier en milieu fortement aqueux, tels que les résorcinols et leurs dérivés, et notamment le composé (S)-4-(2,4-dihydroxyphenyl)-N-(1-phenylethyl)piperidine-1-carboxamide (nommé également A2 dans la présente demande,
- les actifs difficiles à solubiliser et qui nécessitent une forte concentration en solvant organique, tels que les inhibiteurs de TACE (TNFα-converting enzyme) et notamment le composé (S)-N-hydroxy-3-[4-(2-méthyl-quinolin-4-ylméthoxy)-benzènesulfonylamino]-2-[4-(propane-2-sulfonyl)-pipérazin-1-yl]-propionamide (nommé également A3 dans la présente demande),
- les actifs qui, sous forme solubilisée, sont souvent mal tolérés, et dont la dispersion s'avère être une voie favorable pour améliorer leur tolérance, tels que les rétinoïdes et de préférence le Trifarotène (nommé également A1 dans la présente demande), et
- les actifs qui sous forme solubilisée ne présentent pas un profil cinétique de libération contrôlée satisfaisant, tels que les rétinoïdes et de préférence le Trifarotène.

La composition selon l'invention comprend entre 0,00001 et 5 % d'au moins un actif dispersé en poids par rapport au poids total de la composition, de préférence de 0,0001 à 3 % et de manière préférentielle, la composition selon l'invention contient de 0,001 % à 1 % d'un actif en poids par rapport au poids total de la composition.

Selon un mode de réalisation préféré, le principe actif se trouve dispersé dans la phase aqueuse de la composition. Mais l'homme de l'art pourra adapter la phase de dispersion idéale de l'actif au sein de la composition selon sa nature de la composition finale et l'effet recherché.

Les compositions de l'invention permettent d'améliorer la pénétration de principes actifs dispersés, grâce à la présence de microcapsules lipidiques au sein d'un même véhicule pharmaceutiquement acceptable. Dans le cadre de la présente invention, ledit au moins un principe actif dispersé au sein d'un véhicule pharmaceutiquement acceptable se trouve à l'extérieur des microcapsules lipidiques.

La présente invention se rapporte donc à une composition pharmaceutique comprenant au moins un principe actif et des microcapsules lipidiques de taille micrométrique comprenant une phase interne huileuse, et une enveloppe non polymérique obtenue à partir d'au moins un composé lipidique choisi parmi les lipides amphiphiles, dispersés au sein d'un même véhicule pharmaceutiquement acceptable, et dans laquelle les microcapsules lipidiques sont exemptes dudit principe actif.

Par microcapsules lipidiques selon l'invention, on entend des microcapsules lipidiques ayant une phase interne huileuse et une enveloppe non polymérique obtenue à partir d'au moins un composé lipidique choisi parmi les lipides amphiphiles. En effet, par microcapsules lipidiques selon la présente invention, on entend un système vésiculaire de taille micrométrique c'est-à-dire de taille supérieure au micromètre constitué d'une enveloppe lipidique non polymérique entourant un cœur huileux liquide ou semi-liquide à température ambiante.

Par microcapsules lipidiques de taille micrométrique selon la présente invention, on entend plus précisément des microsystèmes lipidiques dont la taille est préférentiellement comprise entre 1 µm et 100 µm.

Selon un mode de fabrication préféré, 50% des microcapsules lipidiques présentent au moins une taille moyenne comprise entre let 80 µm et préférentiellement comprise entre 1 et 50 µm. Dans un mode particulièrement préféré, les microcapsules selon l'invention présentent une taille moyenne comprise entre 1 et 20 µm.

Les microcapsules lipidiques de taille micrométrique sont présentes dans la composition selon l'invention en quantité comprise entre 0,1 et 30% en poids par rapport au poids total de la composition, de préférence entre 0,5 et 20%, et plus particulièrement entre 1 et 10%. L'homme de l'art choisira la quantité de microcapsule adaptée au niveau de pénétration recherché pour un actif dispersé donné.

Les microcapsules sont chacune constituées d'une phase interne lipidique, ou cœur huileux, liquide ou semi-liquide à température ambiante, et d'une enveloppe obtenue à partir d'au moins un composé lipidique.

Par cœur huileux, ou phase interne lipidique, on entend la phase interne des microcapsules lipidiques contenant un composé lipophile non miscible à l'eau ou un mélange de nature lipophile non miscible à l'eau. Ces microcapsules lipidiques et leur procédé d'obtention sont notamment décrits dans les demandes de brevet internationales WO 2015/082659 et WO 2015/082660.

Un des nombreux avantages de la composition selon l'invention est la mise en œuvre des microcapsules lipidiques de taille micrométrique qui ne nécessite pas l'utilisation de solvant organique volatil souvent utilisé pour la formation de l'enveloppe. Ainsi des concentrations moindres, voire une absence de composés solvants au sein de la composition, limitent donc les risques de toxicité et d'intolérance et en particulier d'irritation.

Selon la présente invention, la composition comprend des microcapsules lipidiques de taille micrométrique et non des microsphères lipidiques. Par opposition, les microsphères lipidiques sont des particules matricielles, i.e. dont la totalité de la masse est solide à température ambiante. Les microcapsules lipidiques de taille micrométrique selon l'invention sont des particules dont le cœur est composé d'un ou plusieurs corps gras liquide(s) ou semi-liquide(s) à température ambiante, et dont l'enveloppe est de nature lipidique et non polymérique. En effet, les microcapsules lipidiques de taille micrométrique selon l'invention ne nécessitent aucun polymère et il n'est donc pas observé de polymérisation in situ.

La Demanderesse a donc, de façon surprenante, découvert que des compositions comprenant au moins un principe actif sous forme dispersée, en présence de microcapsules lipidiques de taille micrométrique telles que décrites dans la revendication 1, dans un environnement hydrophile, ne nécessitant pas l'utilisation de polymère ou de solvant organique volatil, garantissaient la stabilité de l'actif et sa pénétration dans la peau même sous forme dispersée au sein de la composition.

Les microcapsules lipidiques de taille micrométrique selon l'invention sont constituées:
- d'une enveloppe non polymérique obtenue à partir d'au moins un composé lipidique amphiphile, et
- d'au moins un cœur huileux.

L'art antérieur (US 8,057,823, FR 2 805 761 et WO2011/036234) présente des capsules lipidiques contenant des phosphatidylcholines mais celles-ci sont de taille nanométrique et nécessitent pour leur élaboration la présence systématique d'au moins un co-surfactant non-ionique hydrophile dérivé oxyéthyléné d'alcools gras et d'acides gras.

D'autres documents de l'art antérieur sont WO 95/03829 A1, US 4 902 513 A, WO 2010/072958 A2, WO 2010/133609 A2.

A la différence de l'art antérieur, la présente invention porte sur des microcapsules lipidiques de taille micrométrique contenant exclusivement des phosphatidylcholines sans aucun autre co-surfactant lipophile ou hydrophile additionnel.

L'enveloppe encapsulant le cœur huileux liquide ou semi-liquide à température ambiante, est de préférence composée d'un matériau rigide à température ambiante, non polymérique et dont la température de transition ou de fusion est élevée. Pour être rigide à température ambiante, la température de transition ou de fusion doit être supérieure à 35°C, de préférence supérieure à 40°C et idéalement supérieure à 45°C.

Dans les microcapsules selon l'invention, l'enveloppe est constituée par au moins un composé lipidique de type amphiphile. Préférentiellement, l'enveloppe est constituée par un seul composé lipidique, avantageusement choisi parmi les lipides amphiphiles. Plus préférentiellement, le composé lipidique est choisi parmi la famille des phospholipides, et plus précisément, des phosphatidylcholines ou lécithines. Les phosphatidylcholines ou lécithines montrent une bonne compatibilité avec la peau avec un très faible potentiel irritant.

Comme lécithines utilisables, on peut citer notamment les lécithines de soja ou d'œuf, naturelles ou synthétiques ou dérivés. Le premier type de lécithine est la phosphatidylcholine (PC). Il existe d'autres types de lécithine incluant le phosphatidylglycerol, le phosphatidylinositol, la sphingomyéline et la phosphatidyléthanolamine.

Parmi les lécithines présentant une température de transition supérieure à 35°C, on peut citer plus particulièrement la dipalmitoylphosphatidylcholine (DPPC), la phosphatidylcholine distéaroyl (DSPC), la phosphatidylcholine dibéhényl (DBPC), la palmitoyl-stéaroyl phosphatidylcholine (PSPC), la palmitoyl-béhényle phosphatidylcholine (PSPC), la stéaroyl-béhényle phosphatidylcholine (SBPC), ainsi que toutes lécithines saturées avec de longues chaines d'acides gras et leurs dérivés.

Les lécithines notamment utilisées dans la présente invention sont solides à température ambiante, ce qui favorise la formation d'une interface semi-solide autour du cœur liquide ou semi-liquide.
Les microcapsules lipidiques de taille micrométrique selon l'invention contiennent plus particulièrement une interface semi-solide ou solide entre la phase interne et la phase continue aqueuse, grâce à l'utilisation comme unique composé lipidique d'une lécithine préférentiellement hydrogénée. Plus particulièrement la lécithine hydrogénée utilisée selon l'invention présente un pourcentage de phosphatidylcholine saturée élevé.
Par pourcentage élevé, on entend une quantité supérieure à 85% de phosphatidylcholine hydrogénée (ou saturée) par rapport au poids total de lécithine.
Comme lécithine préférentiellement utilisées selon l'invention, on peut citer, certaines lécithines hydrogénées avec une teneur en phosphatidylcholine hydrogénée supérieure à 85%, comme par exemple le Lipoid® de grade P 100-3, le Phospholipon® de grade 90H vendus par la société Lipoid, l'Epikuron® de grade 200 SH commercialisé par Cargill, ou l'Emulmetik® 950 vendu par Lucas Meyer. Préférentiellement, la lécithine utilisée comme unique composé lipidique est le Phospholipon® 90H pour lequel la teneur en phosphatidylcholine hydrogénée est supérieure à 90%, dont la température de transition est d'environ 54°C.
Le composé lipidique enveloppant le cœur liquide ou semi-liquide tel que défini ci-dessus est présent en quantité comprise entre 0,01 et 10% en poids, de préférence entre 0,05 et 5% en poids, et plus préférentiellement entre 0,1 et 1% en poids par rapport au poids total de la composition.
En particulier, la microcapsule lipidique, et notamment l'enveloppe, est exempte de tout co-surfactant, en particulier de co-surfactant lipophile ou hydrophile.
Les microcapsules lipidiques de taille micrométrique sont notamment exemptes de solvant organique volatil.
En particulier, les microcapsules lipidiques de taille micrométrique sont exemptes de polymère.

Par lipophile, on entend au sens de la présente invention un composé soluble dans un corps gras liquide ou semi-liquide à température ambiante. En d'autres termes, une substance lipophile est liposoluble.

La composition de la phase interne est donc essentielle pour la pénétration du principe actif. La phase interne huileuse doit bien entendu être susceptible d'être compatible avec l'actif à faire pénétrer. La nature du cœur huileux et la concentration en microcapsules dans la composition influent directement sur le niveau de pénétration du principe actif et est donc choisi afin d'être adapté au mieux à l'actif.
Par compatibilité avec le principe actif dans la phase huileuse, on entend au sens de l'invention que le principe actif est stable chimiquement à température ambiante pendant au moins 24 à 48 h.
Par corps gras liquide ou semi-liquide à température ambiante, on entend au sens de la présente invention un composé lipophile non miscible à l'eau ou un mélange de nature lipophile non miscible à l'eau.
Plus particulièrement, le corps gras liquide ou semi-liquide peut être une huile végétale, minérale, d'origine animale ou synthétique.
De même, la phase interne huileuse peut également contenir en présence d'un corps gras liquide ou semi-liquide, des composés de type solvants organiques non volatils, à condition que le mélange de ces composés présente une solubilité pour l'actif d'intérêt supérieure ou égale à 0,02%.
Parmi les huiles végétales, on peut citer à titre non limitatif l'huile d'olive, l'huile d'amande, l'huile de palme, l'huile de soja, l'huile de sésame, l'huile de canola, l'huile de graines de coton, l'huile de mais, l'huile de carthame, l'huile de ricin ou l'huile de tournesol.
Parmi les huiles minérales, on peut citer à titre non limitatif les huiles de paraffine de différentes viscosités comme par exemple celles vendues par Exxon Mobil, Marcol 152 ®, Marcol 82® et Primol 352®.

Parmi les huiles d'origine animale, on peut citer à titre non limitatif la lanoline, le squalene, l'huile de foie de morue, le squalane vendu par la société Laserson sous le nom commercial Cosbiol®.
Parmi les huiles synthétiques, on peut citer à titre non limitatif, les triglycérides, les acides gras et esters correspondants, les esters d'acides carboxyliques, les alcools gras et esters correspondants, les éthers de polyéthylène glycols, les amides, les huiles silicones Parmi les triglycérides et huiles en contenant, on peut citer à titre non limitatif, les triglycérides d'acide octanoïque ou encore les triglycérides d'acides caprylique/caprique comme ceux vendus par la société Stearineries Dubois ou ceux vendus sous la dénomination Miglyol® 810, 812, et 818 par la société Sasol.
Parmi les acides gras, on peut citer à titre non limitatif l'acide oléique vendu par Croda sous la dénomination de Super refined Oleic acid NF.
Parmi les esters d'acide gras, on peut citer à titre non limitatif le diisopropyl adipate tel le produit commercial Crodamol® DA vendu par la société Croda ou le Schercemol DIA Ester® vendu par la société Lubrizol, ou le cétéaryl isononanoate vendu sous le nom de Cetiol SN® par la société BASF ou Apricot kernel oil PEG-6 esters vendu sous le nom de Labrafil M1944CS® par Gattefossé Parmi les esters d'acides carboxyliques, on peut citer à titre non limitatif le benzoate d'alkyle (C₁₂₋₁₅) tel le produit commercial Crodamol® AB vendu par la société Croda, ou le propylène glycol caprylate vendu sous le nom de Capryol 90® par la société Gattefossé ou encore le C12-C15 alkyl lactate vendu par Ashland sous le nom de Ceraphyl 41.
Parmi les alcools gras, on peut citer à titre non limitatif l'octyl dodécanol, l'octanoate d'octyl dodécanol, l'alcool oléique vendu sous la dénomination Novol par la société Croda.
Parmi les éthers de polyéthylène glycols, on peut citer à titre non limitatif le PPG-15 stéaryl éther vendu sous le nom de Arlamol PS11E-LQ par la société Croda.
Parmi les amides, on peut citer à titre non limitatif le diméthyl capramide vendu sous le nom de Spectrasolv DMDA par Hallstar Company.

Parmi les silicones volatils et non volatils, on peut mentionner les diméthicones et les cyclométhicones, comme celles revendus par Dow Corning sous le nom commercial Q7-9120 silicone fluid® et ST-Cyclomethicone 5-NF®.
Parmi les composés de type solvants organiques non volatils, on peut citer à titre non limitatif le N-méthyl-2-pyrrolidone, le diméthylisosorbide, le diéthylène glycol monoéthyl éther vendu sous le nom de Transcutol HP par la société Gattefossé ou le diméthyl sulfoxide vendu sous le nom de Procipient DMSO par la société Gaylord Chemical Company,
Selon un mode préféré, la phase interne huileuse comprend donc au moins un corps gras, choisi parmi les triglycérides et huiles en contenant, les esters d'acides gras, les acides gras polyéthoxylés, les alcools gras et esters correspondants, les éthers de polyéthylène glycols, les amides et éventuellement un composé de type organique non volatile, choisi parmi diéthylène glycol monoéthyl éther et diméthyl sulfoxide.

En particulier, l'homme du métier choisira le ou les corps gras, et le ou les solvants organiques non volatils, adaptés en fonction de l'actif dont il faut améliorer la pénétration.
Selon un mode de réalisation préféré, les corps gras préférés pour aider à la pénétration dans la peau du Trifarotène sont les triglycérides d'acides caprylique/caprique.
Selon un mode de réalisation préféré, les corps gras préférés pour aider à la pénétration dans la peau du composé (S)-4-(2,4-dihydroxyphenyl)-N-(1-phenylethyl)piperidine-1-carboxamide (A2) sont un mélange triglycérides d'acides caprylique/caprique/diméthyl capramide ou un mélange triglycérides d'acides caprylique/caprique/diéthylène glycol monoéthyl éther ou encore les triglycérides d'acides caprylique/caprique.
Selon un mode de réalisation préféré, le corps gras préféré pour aider à la pénétration dans la peau du composé (S)-N-hydroxy-3-[4-(2-méthyl-quinolin-4-ylméthoxy)-benzènesulfonylamino]-2-[4-(propane-2-sulfonyl)-pipérazin-1-yl]-propionamide (A3) est un mélange de triglycérides / diméthylsulfoxide et de préférence les triglycérides d'acides caprylique/caprique.

Les microcapsules lipidiques contiennent :
- une phase interne huileuse comprenant au moins un corps gras liquide ou semi-liquide à température ambiante ou un mélange corps gras/solvant organique non volatil choisi parmi un triglycéride, un ester d'acide gras, éthers de polyéthylène glycol, diméthyl capramide, diéthylène glycol monoéthyl éther ou diméthylsulfoxide.
- une enveloppe non polymérique obtenue à partir d'au moins un composé lipidique amphiphile.

Dans la phase interne huileuse, le corps gras ou le composé lipophile ou le mélange de nature lipophile non miscible à l'eau sera présent en quantité comprise entre 50 et 99,997% en poids par rapport au poids total de la phase interne ; de préférence en quantité comprise entre 70 et 99,997% en poids par rapport au poids total de la phase interne, de préférence entre 95 et 99,997%.

Dans la phase interne huileuse, le composé de type organique non volatil optionnel est présent en quantité comprise entre 0 et 50% en poids par rapport au poids total de la phase interne ; de préférence en quantité comprise entre 0,1 et 25% en poids par rapport au poids total de la phase interne, de préférence entre 0,5 et 20%.

En sus de ce(s) corp(s) gras et ce(s) composé(s) de type organique(s) non volatil(s), la phase interne peut également comprendre un ou plusieurs composés comme par exemple des antioxydants ou des agents de conservation.

En faisant varier la quantité des microcapsules présentes dans la composition ainsi que la nature du cœur huileux, la présente invention permet de proposer des compositions avec des textures sensorielles différentes tout en assurant l'apport de la richesse souhaitée selon les pathologies à traiter.

La présente invention fait référence également à une émulsion primaire composée des microcapsules lipidiques de taille micrométrique dispersées dans une phase aqueuse.

Par émulsion primaire, on entend donc le système lipidique composé des microcapsules lipidiques de taille micrométrique à interface solide ou semi-solide dispersées dans une phase aqueuse continue, lesdites microcapsules contenant un cœur huileux, une enveloppe obtenue à partir d'un composé lipidique, formant l'interface semi-solide ou solide entre la phase interne huileuse et la phase aqueuse continue. Cette émulsion primaire est donc une émulsion de type huile dans eau.
Ladite émulsion primaire de type huile dans eau selon l'invention peut être incorporée dans un véhicule pharmaceutiquement acceptable, tel un gel, une solution ou une émulsion comme une crème ou une lotion.

Dans l'émulsion primaire selon l'invention, la phase interne huileuse des microcapsules est présente en quantité comprise entre 0,1 et 50% en poids par rapport au poids total de l'émulsion primaire, de préférence en quantité comprise entre 0,5 et 35% en poids par rapport au poids total de l'émulsion primaire.
Dans l'émulsion primaire selon l'invention, le ratio entre la phase huileuse interne et la quantité de lécithine hydrogénée est compris entre 5 à 10 pour 1.
De manière préférée, ce ratio dans l'émulsion est compris entre 6 à 8 pour 1 et préférentiellement 7 pour 1.
Par ailleurs, le ratio entre l'eau et la phase huileuse interne est compris entre 1,25 à 5 pour 1. De manière préférée, ce ratio entre l'eau et la phase huileuse interne est compris entre 2 à 4 pour 1 et préférentiellement 2 à 3 pour 1.
Au sein de l'émulsion primaire, les microcapsules sont dispersées dans une phase aqueuse. La phase aqueuse continue comprend de l'eau. Cette eau peut être de l'eau déminéralisée, une eau florale, ou une eau thermale ou minérale naturelle,
L'eau peut être présente à une teneur comprise entre 55 et 95% en poids par rapport au poids total de la composition, de préférence comprise entre 60 et 95 % en poids.

La présente invention concerne donc également une composition, notamment pharmaceutique, ladite composition comprenant, au sein d'un véhicule pharmaceutiquement acceptable, au moins un principe actif dispersé et l'émulsion primaire telle que définie ci-dessus. La présente invention concerne donc une composition pharmaceutique comprenant au sein d'un véhicule pharmaceutiquement acceptable, au moins un principe actif dispersé en présence de l'émulsion primaire composée de microcapsules lipidiques de taille micrométrique constituées de préférence :
- d'une enveloppe non polymérique obtenue à partir d'au moins un composé lipidique, et
- d'au moins un cœur huileux ;
lesdites microcapsules lipidiques de taille micrométrique étant dispersées dans une phase aqueuse.

Par composition selon l'invention, on entend donc au moins un principe actif dispersé et l'émulsion primaire, incorporés dans un véhicule pharmaceutiquement acceptable, tel un excipient ou un mélange d'excipients pouvant former une composition sous forme d'un gel, une solution ou une émulsion comme une crème ou une lotion pouvant être pulvérisable ou non.

Les compositions selon l'invention présentent l'avantage d'être stable chimiquement et physiquement.

Par stabilité physique selon l'invention, on entend une composition dont les propriétés physiques telles que les caractères organoleptiques, la taille des microcapsules, le pH et la viscosité sont stables au cours du temps et à différentes conditions de températures, 4°C, température ambiante, 40°C.

Par stabilité chimique selon l'invention, on entend une composition capable de contenir un principe actif stable chimiquement au cours du temps et ce quelle que soit la condition de température : 4°C, température ambiante, 40°C.

Par température ambiante, on entend une température comprise entre 15 et 25°C.

La présente invention a donc pour objet une composition, notamment pharmaceutique, ladite composition comprenant le principe actif dispersé et l'émulsion primaire contenant les microcapsules lipidiques de taille micrométrique telles que définies ci-dessus au sein d'un véhicule pharmaceutiquement acceptable, tel un gel, une solution ou une émulsion comme une crème ou une lotion.
Lorsque le véhicule pharmaceutiquement acceptable est un gel, l'émulsion primaire est dispersée dans une phase aqueuse qui comprend au moins un agent gélifiant. De préférence, la phase aqueuse comprend le principe actif est dispersé.
Cet agent gélifiant peut être un dérivé cellulosique choisi parmi les gélifiants cellulosiques semi-synthétiques.
L'agent gélifiant peut également être choisi parmi les gommes naturelles, en particulier la gomme xanthane (connue par exemple sous le nom de Satiaxane et vendu par la société Cargill), l'amidon et ses dérivés, les polymères d'acide polyacrylique réticulés comme les carbomères tels que le Carbopol 980, le Carbopol Ultrez 10 et parmi leurs dérivés alkylés comme les copolymères d'acrylates/C10-30 alkyl acrylate tels que le Carbopol ETD2020, le Pemulen TR1, le Pemulen TR2, les carboxyvinyl polymères, les polyvinyl pyrrolidones et leurs dérivés, les polyvinyl alcools.
L'agent gélifiant peut également être choisi parmi les polymères émulsifiants tels que le Sepigel 305 constitué d'un mélange polyacrylamide/isoparaffine en C13-C14/ laureth-7, ou le Simulgel® 600PHA ou Sepineo® P600, à savoir le sodium acryloyldiméthyltaurate copolymère/ isohexadecane/polysorbate 80. Ces deux produits étant commercialisés par la société Seppic.
Lorsque le véhicule pharmaceutiquement acceptable est une solution, l'émulsion primaire est dispersée au sein d'un véhicule composé d'une phase aqueuse. De préférence, la phase aqueuse comprend le principe actif est dispersé.
Par phase aqueuse qui constitue le véhicule pharmaceutiquement acceptable, on entend toute phase aqueuse telle que définie précédemment dans la présente invention.
Lorsque le véhicule pharmaceutiquement acceptable est une crème ou une lotion, l'émulsion primaire est dispersée au sein d'un véhicule composé d'une phase aqueuse et d'une phase grasse avec la présence optionnelle d'un surfactant ou émulsionnant.
Dans le cas des véhicules pharmaceutiques sous forme crème ou une lotion, la composition selon l'invention comprend donc une phase grasse. Cette phase grasse peut comprendre par exemple, les huiles végétales, minérales, animales ou synthétiques, des huiles de silicones, et leurs mélanges.
De préférence, lorsque le véhicule de la composition selon l'invention est une crème ou une lotion, l'émulsion est sous la forme d'une émulsion huile dans eau (H/E). Cette émulsion peut ne pas comprendre ou comprendre au moins un agent émulsionnant.
La crème ou la lotion selon l'invention comprend également une phase aqueuse.
Par phase aqueuse qui constitue le véhicule pharmaceutiquement acceptable, seule ou au sein d'une émulsion, on entend toute phase aqueuse telle que définie précédemment dans la présente invention.
La composition selon l'invention peut en outre contenir au sein de l'émulsion primaire ou du véhicule pharmaceutiquement acceptable un ou plusieurs additifs ou combinaisons d'additifs, tels que :
- des agents conservateurs;
- des agents propénétrants ;
- des agents stabilisants ;
- des agents humectants ;
- des agents régulateurs d'humidité ;
- des agents régulateurs de pH ;
- des agents modificateurs de pression osmotique ;
- des agents chélatants ;
- des filtres UV-A et UV-B ;
- et des antioxydants.
Bien entendu, l'homme du métier veillera à choisir les ingrédients du véhicule pharmaceutiquement acceptable et notamment, les phases aqueuses, les phases grasses, les émulsionnants ainsi que le ou les éventuels composés à ajouter à ces compositions, de telle manière que les propriétés avantageuses attachées intrinsèquement à la présente invention ne soient pas ou substantiellement pas altérées par le choix des ingrédients. Selon un mode de réalisation préféré, le principe actif se trouve dispersé dans la phase aqueuse de la composition. Mais l'homme de l'art pourra adapter la phase de dispersion idéale de l'actif au sein de la composition selon sa nature de la composition finale et l'effet recherché.

La composition selon l'invention comprend donc, dans un véhicule pharmaceutiquement acceptable, en poids par rapport au poids total de la composition, 0,00001 à 5% d'un actif dispersé en présence de microcapsules composées:
a) d'une enveloppe non polymérique obtenue à partir de 0,01 à 10% de composé lipidique choisi parmi les lipides amphiphiles ;
b) d'un cœur huileux composé de 0,1 à 50% de corps gras et éventuellement des composés de type solvants organiques non volatils tant que le mélange reste lipophile et liquide ou semi-liquide à température ambiante.
Par « actif dispersé en présence de microcapsules », il est entendu que le principe actif est dispersé dans le véhicule pharmaceutiquement acceptable en présence de microcapsules, et non qu'il est compris dans les microcapsules.
La composition selon l'invention comprend ainsi de préférence dans un véhicule pharmaceutiquement acceptable, en poids par rapport au poids total de la composition, 0,0001 à 3% d'un actif dispersé en présence de microcapsules composées de :
a) 0,1 à 5% de composé lipidique choisi parmi les lipides amphiphiles, de préférence la lécithine hydrogénée ;
b) 1 à 30% de corps gras et des composés de type solvants organiques non volatils, de préférence des triglycérides, des esters d'acide gras, des éthers de polyéthylène glycol, du diméthyl isosorbide.
Dans un mode préféré selon l'invention, la composition comprend dans un véhicule pharmaceutiquement acceptable, en poids par rapport au poids total de la composition, 0,001 à 1% d'un actif en présence de microcapsules composées de :
a) 0,1 à 5% de lécithine hydrogénée avec une teneur en phosphatidylcholine hydrogénée supérieure à 85% ;
b) 1 à 30% de corps gras et des composés de type solvants organiques non volatils, de préférence des triglycérides, des esters d'acide gras, des éthers de polyéthylène glycol, du diméthyl isosorbide.

La composition pharmaceutique utilisable selon l'invention est destinée au traitement de la peau et peut être administrée par voie topique, parentérale ou orale.
Par voie orale, la composition pharmaceutique peut se présenter sous forme liquide, ou pâteuse, et plus particulièrement sous formes de gélules, de dragées, ou de sirops.
Par voie parentérale, la composition peut se présenter sous forme de suspensions pour perfusion ou pour injection.
De préférence, la composition se présente sous une forme adaptée pour une administration par voie topique. Par voie topique, on entend une application sur la peau, les muqueuses, les cheveux ou le cuir chevelu.
Par voie topique, la composition peut se présenter sous forme liquide, ou pâteuse, et plus particulièrement sous forme de crèmes, de laits, de pommades, de tampons imbibés, de syndets, de lingettes, de gels, de sprays, de mousses, de lotions, de sticks, de shampoings, ou de bases lavantes.

L'invention a également pour objet un procédé de préparation des compositions selon l'invention. Les procédés de préparations de la composition de l'invention et des microparticules sont notamment décrits dans les demandes de brevets internationales WO 2015/082659 et WO 2015/082660, incorporées ici par référence.

Le procédé selon l'invention ne met pas en jeu des phénomènes d'inversion de phase caractérisés par une Température d'Inversion de Phase (TIP) (utilisé notamment dans les brevets FR 2 805 761 et FR 2 840 531), et ne nécessite donc pas de cycle(s) de montée et de descente en température.
Le procédé selon l'invention n'utilise pas d'homogénéisateur haute pression (HHP) et donc ne nécessite pas d'étape de pré-homogénéisation.

Le procédé selon l'invention présente donc l'avantage à la fois de ne pas présenter des cycles de chauffage et de refroidissement successifs, de ne pas utiliser de solvant organique volatil, de polymère, de ne pas nécessiter d'étape de gélification de l'émulsion et ni d'étape de pré-homogénéisation.
Le procédé tel que présenté selon l'invention et proposé pour la réalisation des microcapsules lipidiques de taille micrométrique utilise des équipements permettant une émulsification à haut taux de cisaillement.
Différents appareils peuvent être utilisés comme par exemple les mélangeurs de type rotor/stator à haut cisaillement tel qu'un Polytron (Kinematica) ou le Magic Lab (Ika). De façon également alternative au rotor/stator, la sonication peut être utilisée avec par exemple une sonde de type Branson. Quel que soit le type d'équipement utilisé, le procédé consiste à réaliser une émulsion primaire, diluée ensuite dans un véhicule pharmaceutiquement acceptable.
Cette émulsion primaire permet de faire varier le mode d'introduction du composé lipidique, de préférence de la lécithine hydrogénée, qui peut être introduite totalement dans la phase huileuse (100% phase huileuse) ou dans la phase aqueuse (100% phase aqueuse) ou introduite sous différents rapports comme par exemple un rapport 50/50 dans la phase huileuse et dans la phase aqueuse.

### 1 - Préparation de l'émulsion primaire :

La réalisation de l'émulsion primaire comprend 3 étapes :
- Préparation de la phase aqueuse
- Préparation de la phase huileuse
- Mélange des phases aqueuse et huileuse

La préparation de la phase aqueuse et de la phase huileuse sont dépendantes du choix du mode de dispersion du composé lipidique, de préférence de la lécithine hydrogénée :
- 100 % en phase aqueuse ou
- 100% en phase huileuse ou
- 50/50% phase aqueuse / phase huileuse.

a) Préparation de l'émulsion primaire avec dispersion à 100% du composé lipidique, de préférence de la lécithine hydrogénée dans la phase aqueuse :

### Préparation de la phase aqueuse :

Dans un récipient adapté pour contenir la totalité de l'émulsion primaire, la lécithine hydrogénée mise en œuvre est dispersée dans la totalité de la phase aqueuse chauffée à environ 75°C, à l'aide d'un mélangeur de type rotor/stator à haut cisaillement tel qu'un Ultra Turrax (Ika), un Polytron (Kinematica) ou le Magic Lab (Ika), sous agitation comprise entre 5 000 à 10 000 t/min, pendant un temps défini qui ne dépassera pas 30 minutes. Un conservateur et un antioxydant peuvent être ajoutés à cette phase.

### Préparation de la phase huileuse :

Dans un récipient adapté et à l'aide d'un barreau magnétique, les composés du mélange lipophile seront mélangés et chauffés à 75°C. Un conservateur et un antioxydant peuvent être ajoutés à cette phase.

b) Préparation de l'émulsion primaire avec dispersion à 100% du composé lipidique, de préférence de la lécithine hydrogénée dans la phase huileuse :

### Préparation de la phase aqueuse :

Dans un récipient adapté pour contenir la totalité de l'émulsion primaire, la totalité de la phase aqueuse est chauffée à 75°C. Un conservateur et un antioxydant peuvent être ajoutés à cette phase.

### Préparation de la phase huileuse :

Dans un récipient adapté et à l'aide d'un barreau magnétique, les composés du mélange lipophile seront mélangés et chauffés à 75°C. Un conservateur et un antioxydant peuvent être ajoutés à cette phase. Le composé lipidique, de préférence la lécithine hydrogénée mise en œuvre est dispersée dans cette phase huileuse toujours à environ 75°C, à l'aide d'un mélangeur de type rotor/stator à haut cisaillement tel qu'un Ultra Turrax (Ika), ou un Polytron (Kinematica) sous agitation comprise entre 5 000 à 10 000 t/min, pendant un temps défini qui ne dépassera pas 30 minutes.

c) Préparation de l'émulsion primaire avec dispersion de la lécithine hydrogénée 50% dans la phase aqueuse et 50% dans la phase huileuse :

### Préparation de la phase aqueuse :

Dans un récipient adapté pour contenir la totalité de l'émulsion primaire, la totalité de la phase aqueuse est chauffée à 75°C. Environ la moitié du composé lipidique, de préférence de la lécithine hydrogénée mise en œuvre est dispersée dans cette phase aqueuse toujours chauffée à environ 75°C, à l'aide d'un mélangeur de type rotor/stator à haut cisaillement tel qu'un Ultra Turrax (Ika), un Polytron (Kinematica), ou le Magic Lab (Ika) sous agitation comprise entre 5 000 à 10 000 t/min, pendant un temps défini qui ne dépassera pas 30 minutes. Un conservateur et un antioxydant peuvent être ajoutés à cette phase.

### Préparation de la phase huileuse :

Dans un récipient adapté et à l'aide d'un barreau magnétique, les composés du mélange lipophile seront mélangés et chauffés à 75°C. L'autre partie du composé lipidique, de préférence de la lécithine hydrogénée, est dispersée dans cette phase huileuse toujours chauffée à environ 75°C, à l'aide d'un mélangeur de type rotor/stator à haut cisaillement tel qu'un Ultra Turrax (Ika) ou un Polytron (Kinematica), sous agitation comprise entre 5 000 à 10 000 t/min, pendant un temps défini qui ne dépassera pas 30 minutes. Un conservateur et un antioxydant peuvent être ajoutés à cette phase.

Une fois les phases aqueuse et huileuse préparées, celles-ci sont mélangées par incorporation de la phase huileuse dans la phase aqueuse. Le mode opératoire est dépendant du type d'appareil utilisé. Trois types d'appareil sont préférentiellement utilisés pour réaliser le mélange des deux phases résultant en l'émulsion primaire selon l'invention: le procédé avec Polytron, le procédé avec Magic Lab, le procédé avec sonde de sonication. Selon les différents types d'agitateurs, la réalisation de l'émulsion se fait comme décrit :
- Procédé avec Polytron sous régulation de la température à 75°C:
   - Incorporation de la phase huileuse sur la phase aqueuse doucement, sous agitation comprise entre 5000 à 10000 t/min
   - Une fois l'incorporation réussie, agitation à une vitesse plus forte pendant minimum 30 minutes.
- Procédé avec Magic Lab sous régulation de la température à 75°C :
   - Incorporation simultanée de la phase aqueuse et de la phase huileuse dans l'appareil sous agitation à une vitesse inférieure à 16 000 t/min si le composé lipidique, de préférence la lécithine hydrogénée, a été dispersé à 100% dans la phase grasse.
   - Incorporation de la phase huileuse sur la phase aqueuse déjà présente dans l'appareil sous agitation à une vitesse inférieure à 16 000 t/min si le composé lipidique, de préférence la lécithine hydrogénée a été dispersé à 100% dans la phase aqueuse.
   - Une fois l'incorporation réussie, laisser circuler le mélange jusqu'au retour à température ambiante.
- Procédé avec la sonde de sonication avec régulation de la température fixée inférieure à 50°C:
   - Incorporation de la phase huileuse sur la phase aqueuse rapidement, à une amplitude des ultra- sons fixée à 80 microns,
   - Laisser le mélange pendant plusieurs dizaines de secondes sous ces conditions.

### 2 - Réalisation de la composition finale selon l'invention

L'émulsion primaire obtenue est introduite dans un véhicule pharmaceutiquement acceptable préalablement réalisé contenant le principe actif dispersé, de type solution, crème, lotion et gel.

Dans le cas d'un gel contenant principalement que de l'eau et un gélifiant, l'étape de gélification se réalise instantanément à la fin de la fabrication de l'émulsion primaire :
- Prélever une quantité déterminée d'émulsion primaire et
- L'incorporer doucement dans un gel préalablement réalisé, sous agitation douce. L'agitation peut être générée par l'utilisation d'une pâle défloculeuse fixée sur un moteur d'agitation de type IKA ou Rayneri. Une agitation douce correspond à une vitesse qui permet d'obtenir un gel homogène au bout de 20 min sans générer une aération trop importante de la formulation, par exemple une vitesse autour de 200 rpm.
De façon alternative, pour réaliser une composition de type gel selon l'invention, une quantité d'émulsion primaire peut être prélevée puis diluée dans une part d'eau. Ce mélange est ensuite épaissi par l'ajout d'un agent gélifiant.

Le procédé de préparation des compositions selon l'invention comprend les étapes suivantes :
(i) Préparation de l'émulsion primaire par :
   (a) Préparation de la phase huileuse ;
   (b) Préparation de la phase aqueuse ;
   (c) Dispersion du composé lipidique dans la phase huileuse obtenue en (a) ou dans la phase aqueuse obtenue en (b) ou en partie au sein de chacune des phases huileuse et aqueuse,
   (d) Chauffage des deux phases huileuse et aqueuse séparément à environ 75°C,
   (e) Mélange sous agitation des phases huileuse et aqueuse obtenues à l'issue de l'étape (d) ;
(ii) Incorporation de la composition obtenue à l'étape précédente dans un véhicule pharmaceutiquement acceptable comprenant au moins un principe actif dispersé.

La demanderesse a découvert de manière surprenante que le mode d'introduction du composé lipidique, et plus particulièrement de la lécithine hydrogénée, était susceptible d'avoir une influence sur la stabilité dans le temps des microcapsules dispersées au sein du véhicule pharmaceutiquement acceptable.

De préférence, le composé lipidique est introduit soit à 100% dans la phase huileuse, soit à 100% dans la phase aqueuse en fonction de la nature du cœur huileux choisi pour aider à la pénétration du principe actif dispersé au sein du véhicule pharmaceutiquement acceptable. Plus préférentiellement, la lécithine hydrogénée est introduite soit à 100% dans la phase huileuse, soit à 100% dans la phase aqueuse selon la nature du cœur huileux choisi. Dans un mode préféré selon l'invention, l'appareil préféré est le Magic Lab.

Dans un mode préféré selon l'invention, le mode de dispersion préféré du composé lipidique, et plus préférentiellement de la lécithine hydrogénée est à 100% dans la phase grasse, dans le cas d'utilisation de composés huileux de type triglycérides et esters d'acides comme par exemple le diisopropyl adipate.

Dans un autre mode préféré selon l'invention, le mode de dispersion préféré du composé lipidique, et plus préférentiellement de la lécithine hydrogénée, est à 100% dans la phase aqueuse notamment dans le cas d'utilisation de composés huileux de type éthers de polyéthylène glycols comme par exemple le PPG-15-stéaryl éther.

En particulier, l'homme du métier choisira le cœur huileux adapté en fonction du principe actif dispersé dans la composition finale, afin d'améliorer au mieux la pénétration de celui-ci. L'homme de l'art adaptera également le mode de dispersion préféré du composé lipidique, et plus préférentiellement de la lécithine hydrogénée.
Dans un des modes préférés, le procédé de préparation d'une composition selon l'invention comprend les étapes suivantes :
(i) préparation de l'émulsion primaire par :
   a) préparation d'une phase huileuse chauffée à 75°C.
   b) préparation d'une phase aqueuse, chauffée à 75°C
   c) incorporation simultanée de la phase aqueuse et de la phase huileuse dans l'appareil sous agitation à une vitesse inférieure à 16 000 t/min
   d) une fois l'incorporation réussie, laisser circuler le mélange jusqu'au retour à température ambiante.
(ii) incorporation de l'émulsion primaire dans le véhicule pharmaceutiquement acceptable contenant le principe actif dispersé.

Dans un des modes préférés, le procédé de préparation d'une composition selon l'invention comprend les étapes suivantes :
(i) préparation de l'émulsion primaire par :
   a) préparation d'une phase huileuse chauffée à 75°C.
   b) dispersion du composé lipidique, et plus préférentiellement de la lécithine hydrogénée, dans une phase aqueuse, chauffée à 75°C
   c) incorporation de la phase huileuse sur la phase aqueuse déjà présente dans l'appareil sous agitation à une vitesse inférieure à 16 000 t/min.
   d) une fois l'incorporation réussie, laisser circuler le mélange jusqu'au retour à température ambiante.
(ii) incorporation de l'émulsion primaire dans le véhicule pharmaceutiquement acceptable contenant le principe actif dispersé
De préférence, ces procédés de préparation sont mis en œuvres en l'absence de solvant organique volatil.

Comme indiqué précédemment, la composition selon l'invention comprend au sein d'un véhicule pharmaceutiquement acceptable, au moins un principe actif dispersé en présence de microcapsules lipidiques de taille micrométrique dispersées dans une phase aqueuse, lesdites microcapsules lipidiques de taille micrométrique contenant une phase interne huileuse, et une enveloppe non polymérique obtenue à partir d'au moins un composé lipidique choisi parmi les lipides amphiphiles.

Dans un mode préféré selon l'invention, la composition contenant des microcapsules permet d'augmenter la pénétration de l'actif dispersé. Préférentiellement, la pénétration dans la peau d'un actif dispersé en présence de microcapsules lipidiques par rapport à celle obtenue avec un actif dispersé sans microcapsules est supérieure d'un facteur de 2 à 50 fois, de préférence de 2 à 20 fois.

La présente invention porte donc sur l'utilisation de la composition selon l'invention pour améliorer la pénétration d'un actif dispersé dans la peau.

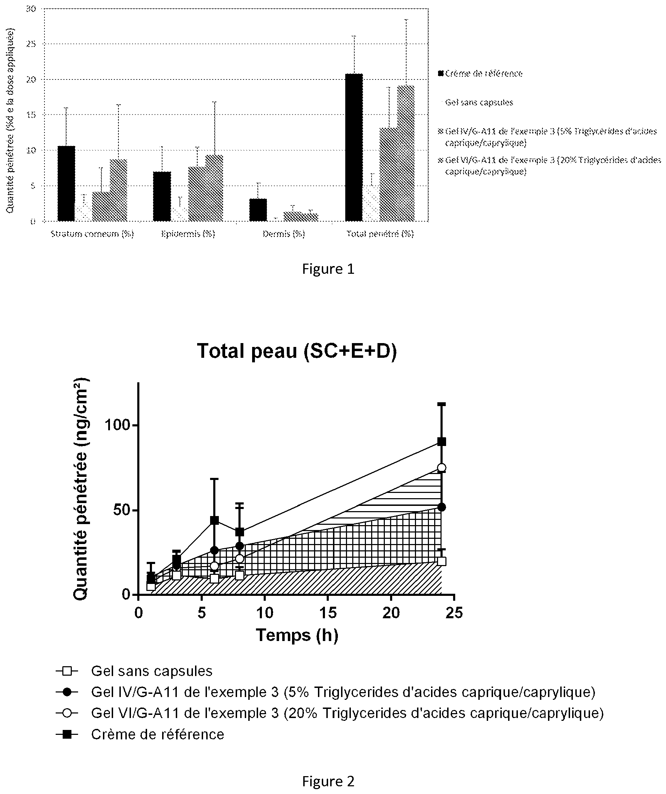

Dans un autre mode, la présente invention concerne l'utilisation de la composition pour améliorer le profil cinétique de pénétration d'un actif dispersé dans la peau. Tel que démontré dans les exemples non imitatifs qui suivront (cf. Ex. 6 et Fig.2), le profil cinétique de pénétration de l'actif dispersé au sein de la composition selon l'invention se rapproche sur des temps longs de celui observé avec une composition dans laquelle le principe actif est solubilisé. L'avantage particulier de la composition selon l'invention contenant l'actif dispersé est que l'exposition est diminuée sur les temps courts (5h) qui induit donc une meilleure tolérance.

La composition selon l'invention contenant les microcapsules lipidiques confère donc une meilleure pénétration de l'actif dispersé, comparé à une composition similaire sans microcapsules et, se rapproche du niveau de pénétration de l'actif sous sa forme solubilisés tout en montrant un profil cinétique améliorant la tolérance.

La composition selon l'invention est utilisable comme médicament.

En particulier, l'invention a également pour objet la composition telle que définie précédemment pour son utilisation pour traiter les affections dermatologiques, notamment humaines, telles que définies ci-après :
1) les affections dermatologiques liées à un désordre de la kératinisation portant sur la différenciation et sur la prolifération cellulaire notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle ;
2) les troubles de la kératinisation, notamment les ichtyoses, les états ichtyosiformes, l'ichtyose lamellaire, la maladie de Darrier, les kératodermies palmoplantaires, les leucoplasies, le pityriasis rubra pilaire et les états leucoplasiformes, le lichen cutané ou muqueux (buccal) ;
3) les affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore la dermatite atopique et les différentes formes d'eczéma;
4) les désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique, telle la xérose, les pigmentations et les rides ;
5) Toute condition liée à des proliférations dermiques ou épidermiques bénignes, qu'elles soient ou non d'origine virale telles que verrues vulgaires, les verrues planes, le molluscum contagiosum et l'épidermodysplasie verruciforme, les papillomatoses orales ou florides ;
6) les désordres dermatologiques tels que les dermatoses immunes comme le lupus érythémateux, les maladies immunes bulleuses et les maladies du collagène, telle la sclérodermie ;
7) les stigmates de l'atrophie épidermique et/ou dermique induite par les corticostéroïdes locaux ou systémiques, ou toute autre forme d'atrophie cutanée,
8) les troubles de la cicatrisation, ou pour prévenir ou pour réparer les vergetures, ou encore pour favoriser la cicatrisation,
9) dans le traitement de toute affection d'origine fongique au niveau cutané tel que le tinea pedis et le tinea versicolor,
10) les désordres de la pigmentation, tel l'hyperpigmentation, le mélasma, l'hypopigmentation ou le vitiligo ;
11) les états cancéreux ou précancéreux, cutanés ou muqueux comme les kératoses actiniques, la maladie de Bowen, les carcinomes in-situ, le kératoacanthome et les cancers cutanés comme le carcinome basocellulaire (BCC), le carcinome spinocellulaire (SCC) et les lymphomes cutanés tek que le lymphome T.
Préférentiellement, l'invention porte sur la composition pour son utilisation dans le traitement de l'acné, les ichtyoses, les états ichtyosiformes, l'hyperkératose palmoplantaire ou le psoriasis.
Autrement dit, l'invention porte sur la composition selon l'invention pour son utilisation comme médicament dans le traitement des affections dermatologiques, notamment humaines, telles que précédemment définies.
De manière particulièrement préférée la composition selon l'invention est utilisée pour le traitement de l'acné, les ichtyoses, les états ichtyosiformes, l'hyperkératose palmoplantaire, la rosacée ou le psoriasis.

Il va être maintenant donné, à titre d'illustration et sans aucun caractère limitatif, diverses formulations de compositions comprenant des microcapsules lipidiques au sein d'un véhicule pharmaceutiquement acceptable et des compositions avec au moins un principe actif dispersé en présence de microcapsules lipidiques.

### EXEMPLES

### Exemple 1: Emulsions primaires contenant les microcapsules lipidiques avant dilution

En utilisant les procédés de préparation cités auparavant et selon le mode de dispersion de la lécithine hydrogénée tel que défini précédemment dans la présente description, des microcapsules lipidiques ont été réalisées avec un cœur huileux contenant une huile (cas des émulsions primaires I à VI) ou un mélange d'huiles (cas des émulsions primaires VII à IX).

Les compositions des émulsions primaires I à IX sont donc les suivantes :

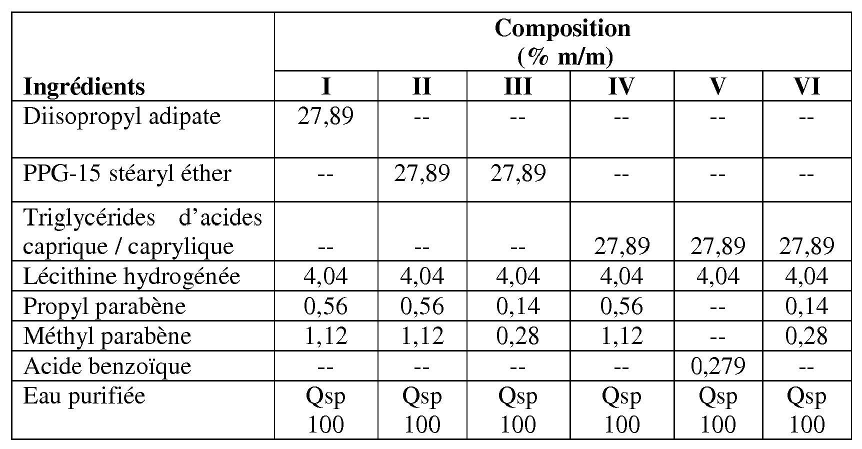

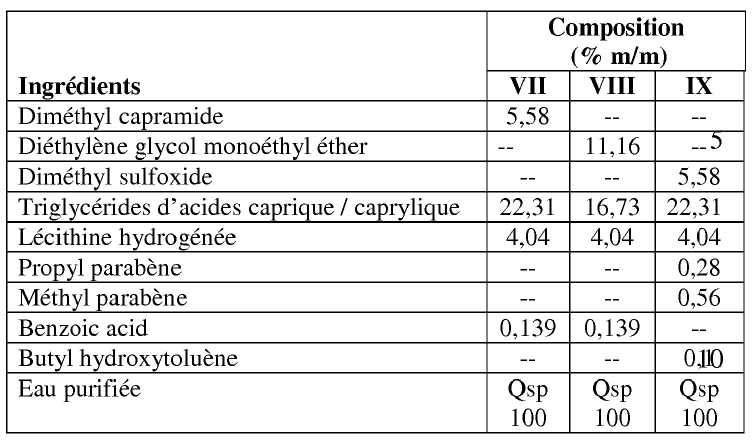

### Exemple 2 : Données de solubilité et de stabilité du Trifarotène dans différents corps gras

La solubilité et stabilité de l'actif a été évaluée par chromatographie en phase liquide couplée à un détecteur UV (HPLC-UV).

| Nom INCI (nom commercial) | Solubilité maximale (%m/m) (TA) | Stabilité |
|---|---|---|
| Propylène glycol monocaprylate (Capryol® 90) | 0,802 | 6 mois TA/40°C |
| Propylène glycol monolaurate (Lauroglycol® FCC) | 0,296 | 6 mois TA/40°C |
| Diisopropyl adipate (Schercemol Dia Ester) | 0,297 | 6 mois TA/40°C |
| PPG-15 stéaryl éther (Arlamol PS11E-LQ) | 0,292 | 6 mois TA/40°C |
| Oléate de macrogol (Labrafil® M1944CS) | 0,156 | 6 mois TA/40°C |
| Octvl dodécanol (Eutanol® G) | 0,137 | Instable |
| Propylène glycol dicaprylate/dicaprate (Myritol® PC) | 0,069 | Instable |
| Alkyl (C12-15) benzoate (Crodamol AB) | 0,026 | Non suivi |
| Triglycérides d'acides caprique / caprylique / (Miglyol® 812N) | 0,019 | 6 mois TA/40°C |
| Huile d'amande douce | 0,011 | 6 mois TA/40°C |
| Huile minérale | 0,0001 | Non suivi |

Suite aux résultats de cette étude de solubilité et de stabilité, on constate que le propylène glycol monocaprylate, le propylène glycol monolaurate, le diisopropyl adipate, le PPG-15 stéaryl éther et l'oléate de macrogol sont adaptés pour solubiliser le Trifarotène.

Suite à ces résultats, le diisopropyl adipate et le PPG-15 stéaryl éther sont les solvants préférés choisis pour être insérés au sein du cœur huileux dans les microcapsules.
Les triglycérides d'acides caprique/caprylique restent le composé huileux choisi parmi les moins bons solvants pour être insérés au sein du cœur huileux dans les microcapsules.

### Exemple 3: Exemples de compositions de type gel selon l'invention contenant le Trifarotène (A1) dispersé en présence de microcapsules lipidiques réalisées à partir des émulsions primaires de l'exemple 1

Afin de réaliser des compositions de type gel I/G-A1₁, II/G-A1₁, III/G-A1₁, IV/G-A1₁ et VI/G-A1₁ selon l'invention, une quantité d'émulsion primaire correspondante préparée selon l'exemple 1 a été prélevée et diluée dans une base gel.

Pour obtenir 100 grammes de gel contenant environ 5% d'huile répartie au sein des microcapsules, 17,784 grammes d'émulsion primaire est ajoutée dans une formulation contenant 0,01% de Trifarotène micronisé dispersé (cas du gel I/G-A1₁, II/G-A1₁ et IV/G-A1₁).

Pour obtenir 100 grammes de gel contenant environ 20% d'huile répartie au sein des microcapsules, 71,71 grammes d'émulsion primaire est ajoutée dans une formulation contenant 0,01% de Trifarotène micronisé dispersé (cas du gel III/G-A1₁ et VI/G-A1₁).

Les émulsions primaires I, II, III, IV et VI conduisent respectivement aux compositions gels I/G-A1₁, II/G-A1₁, III/G-A1₁, IV/G-A1₁ et VI/G-A1₁ décrites dans le tableau ci-après.

Des exemples de compositions de type gel obtenues selon l'invention sont donc les suivants :

| **Ingrédients** | **Composition (% m/m)** | | | | |
|---|---|---|---|---|---|
| | **I/G-A1₁** | **II/G-A1₁** | **III/G-A1₁** | **IV/G-A1₁** | **VI/G-A1₁** |
| Trifarotène micronisé | 0,01 | 0,01 | 0,01 | 0,01 | 0,01 |
| Diisopropyl adipate | 4,96 | -- | -- | -- | -- |
| PPG-15 stéaryl éther | -- | 4,96 | 20 | -- | -- |
| Triglycérides d'acides caprique / caprylique | -- | -- | -- | 4,96 | 20 |
| Lécithine hydrogénée | 0,72 | 0,72 | 2,90 | 0,72 | 2,90 |
| Méthyl parabène | 0,2 | 0,2 | 0,2 | 0,2 | 0,2 |
| Propyl parabène | 0,1 | 0,1 | 0,1 | 0,1 | 0,1 |
| Sodium acryloyldiméthylt aurate copolymère/ isohexadécane/po lysorbate 80 | 4 | 4 | 4 | 4 | 4 |
| Sodium docusate | 0,05 | 0,05 | 0,05 | 0,05 | 0,05 |
| Disodium edetate | 0,1 | 0,1 | 0,1 | 0,1 | 0,1 |
| Glycérol | 4 | 4 | 4 | 4 | 4 |
| Propylène glycol | 4 | 4 | 4 | 4 | 4 |
| Poloxamer P124 | 0,2 | 0,2 | 0,2 | 0,2 | 0,2 |
| Acide lactique (qs pH 3,5-4) | Qsp pH | Qsp pH | Qsp pH | Qsp pH | Qsp pH |
| Eau purifiée | Qsp 100 | Qsp 100 | Qsp 100 | Qsp 100 | Qsp 100 |

### Exemple 4: Caractérisation des compositions de l'exemple 3 de type gel selon l'invention, selon le composé lipophile mis en œuvre et le pourcentage de mélange lipophile ou de nature lipophile réparti dans les microcapsules

Dans les présents exemples, l'équipement qui a été utilisé pour la réalisation des émulsions primaire est le Magic Lab.
Le mode de dispersion préféré pour la lécithine hydrogénée avec le diisopropyl adipate est 100% dans la phase grasse.
Le mode de dispersion préféré pour la lécithine hydrogénée avec le PPG-15 stéaryl éther est 100% dans la phase aqueuse.
Le mode de dispersion préféré pour la lécithine hydrogénée avec les triglycérides d'acides caprique/caprylique est 100% dans la phase grasse.

Les compositions gels I/G-A1₁, II/G-A1₁ et IV/G-A1₁ contiennent 0.01% Trifarotène micronisé dispersé, en présence d'environ 5% d'huile répartie au sein des microcapsules.

| Emulsion primaire / Huile | Caractérisations | Résultats |
|---|---|---|
| **Composition I/G-A1₁** | | |
| I/ Diisopropyl adipate | Observation macroscopique | Gel blanc |
| | Observation microscopique | Capsules de taille micrométrique et présence de cristaux de Trifarotène |
| | pH | 4.99 |
| | Viscosité RV, S06, 10 rpm | 66 300 cP |

| **Composition II/G-A1₁** | | |
|---|---|---|
| II/ PPG-15 stéaryl éther | Observation macroscopique | Gel blanc |
| | Observation microscopique | Capsules de taille micrométrique et présence de cristaux de Trifarotène |
| | pH | 4.67 ou 4.76 |
| | Viscosité RV, S06, 10 rpm | 64 900 cP |

| **Composition IV/G-A1₁** | | |
|---|---|---|
| IV/ Triglycérides d'acides caprique/caprylique | Observation macroscopique | Gel blanc |
| | Observation microscopique | Capsules de taille micrométrique et présence de cristaux de Trifarotène |
| | pH | 4.82 |
| | Viscosité RV, S06, 10 rpm | 60 300 cP |

Les compositions gels III/G-A1₁ et VI/G-A1₁ contiennent 0.01% Trifarotene micronisé dispersé, en présence d'environ 20% d'huile répartie au sein des microcapsules.

| | | |
|---|---|---|
| Emulsion primaire / Huile | Caractérisations | Résultats |

| **Composition III/G-A1₁** | | |
|---|---|---|
| III/ PPG-15 stéaryl éther | Observation macroscopique | Gel blanc |
| | Observation microscopique | Capsules de taille micrométrique et présence de cristaux de Trifarotène |
| | pH | 5.02 |
| | Viscosité RV, S06, 2.5 rpm | 330 000 cP |

| **Composition VI/G-A1₁** | | |
|---|---|---|
| VI/ Triglycérides d'acides caprique/caprylique | Observation macroscopique | Gel blanc |
| | Observation microscopique | Capsules de taille micrométrique et présence de cristaux de Trifarotène |
| | pH | 4.82 |
| | Viscosité RV, S06, 2.5 rpm | 370 000 cP |

### Exemple 5 : Etude de stabilité des gels de l'exemple 4 selon le composé lipophile mis en œuvre et selon le pourcentage de mélange lipophile ou de nature lipophile réparti dans les microcapsules

Gel obtenu à partir de la composition **I/G-A1₁** de l'exemple 3 (4.96% Diisopropyl adipate)

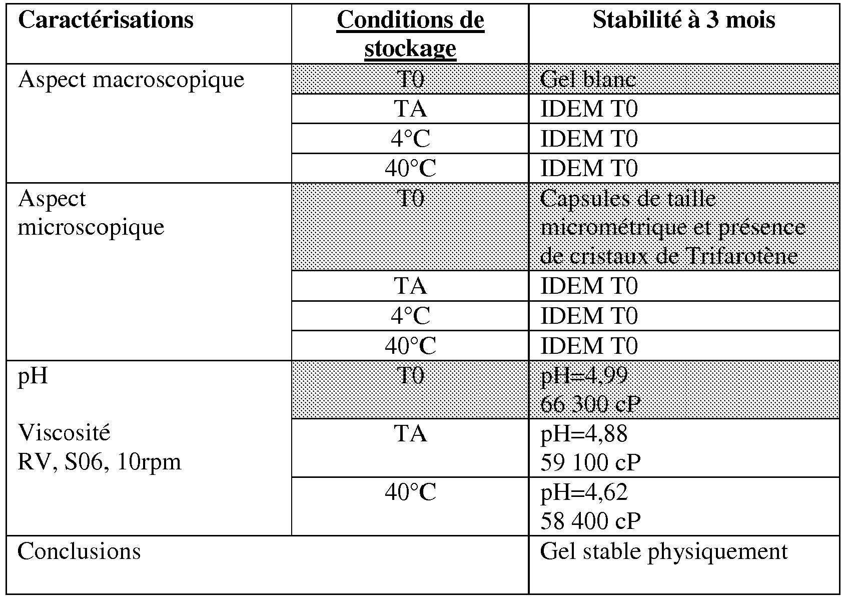

Gel obtenu à partir de la composition **II/G-A1₁** de l'exemple 3 (4.96% PPG15 stéaryl éther)

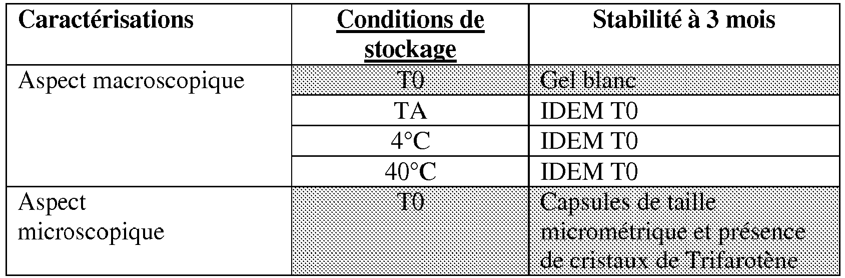

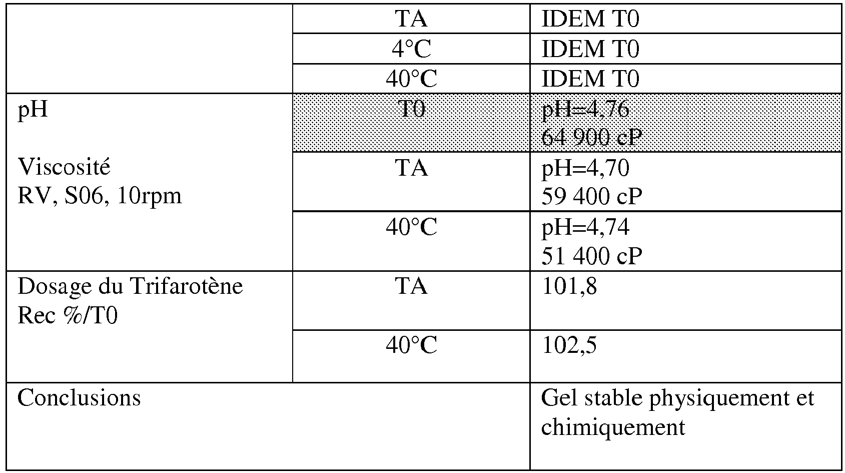

Gel obtenu à partir de la composition **IV/G-A1₁** de l'exemple 3 (4.96% Triglycérides d'acides caprique/caprylique)

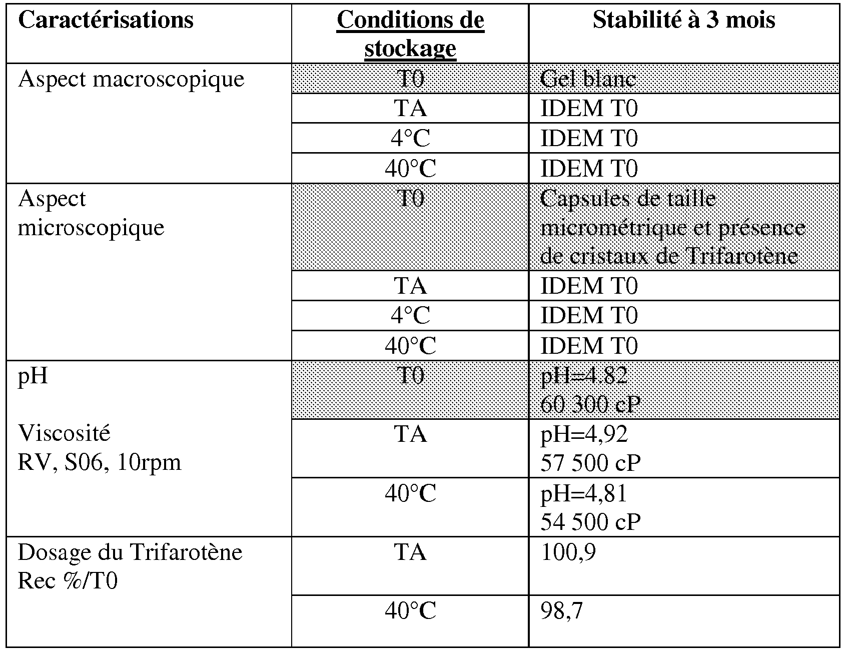

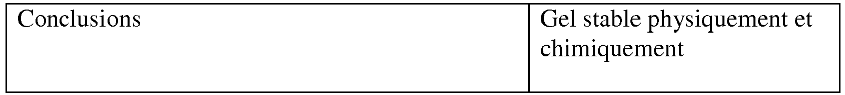

Gel obtenu à partir de la composition **III/G-A1₁** de l'exemple 3 (20% PPG15 stéaryl éther)

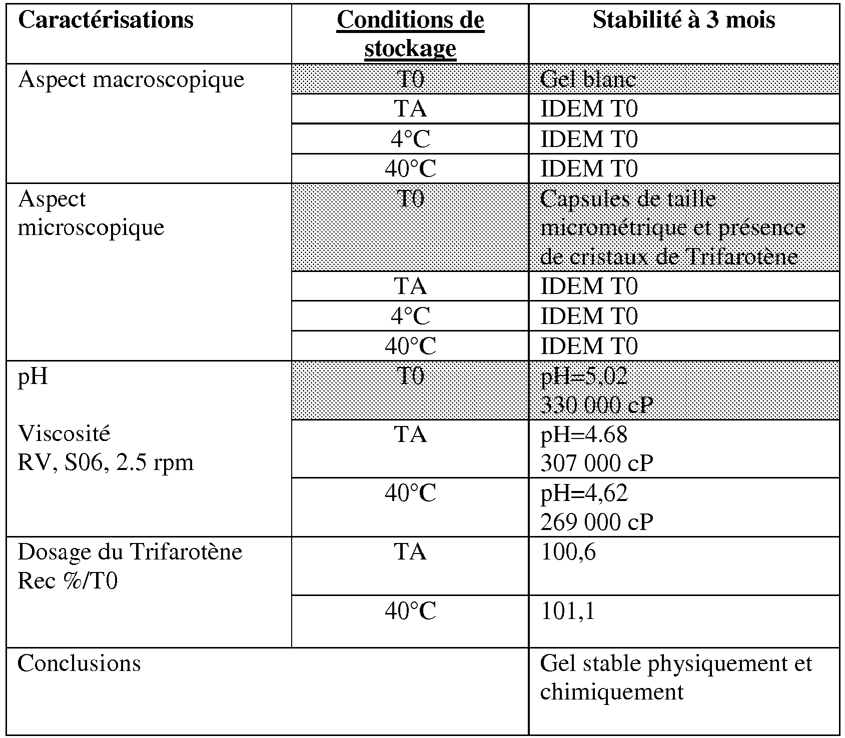

Gel obtenu à partir de la composition **VI/G-A1₁** de l'exemple 3 (20% Triglycérides d'acides caprique/caprylique)

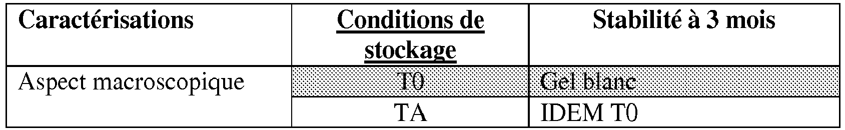

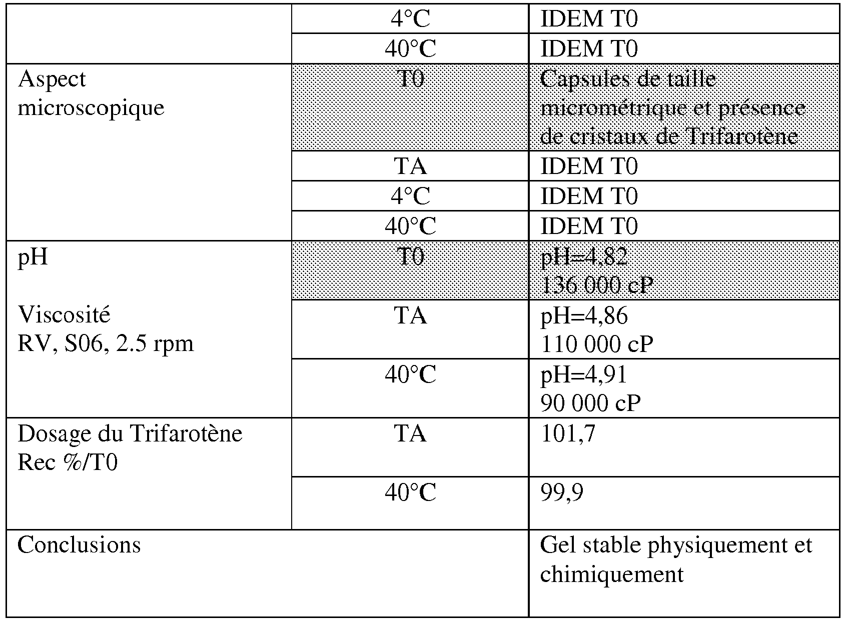

Les résultats montrent que l'on obtient des gels stables à trois mois à température ambiante et à 40°C en présence d'un principe actif dispersé, à savoir le Trifarotène.

### Exemple 6: Etude de pénétration cinétique cutanée in vitro du Trifarotène dispersé dans des formulations gels en présence de microcapsules réalisées selon l'exemple 1

### Conditions de l'étude :

Dans cette étude, les formulations ont été appliquées pendant 24 heures à la surface de la peau. Après application, le Trifarotène est quantifié à plusieurs temps (1h ; 3h ; 6h ; 8h et 24h) dans les différents compartiments de la peau : stratum corneum, épiderme, derme et liquide récepteur selon une méthode de bioanalyse validée réalisée par spectrométrie de masse en tandem par ionisation électrospray positive, et utilisant un appareil Xevo (Waters). La limite de quantification pour le Trifarotène est de 0,01 ng/mL. Les conditions de LC/MS/MS mises au point ont permis de détecter jusqu'à 0,1% de la dose appliquée dans chacun des compartiments (dose non absorbée, stratum, épiderme, derme et liquide récepteur).

Les détails de l'application cutanée sont donnés dans le tableau ci-dessous.

| Peau : 3 donneurs, 2 échantillons par donneurs | |
|---|---|
| Source | Peau humaine abdominale entière |
| Cellules de Franz | 1 cm² |
| Volume liquide récepteur | 3mL |
| Fonction barrière | Evaluée par détermination de la Perte Insensible en Eau, acceptable sauf contre-indication |
| Crème de référence contenant 100 µg/g Trifarotène solubilisé | |
| Gel de référence contenant 100 µg/g Trifarotène micronisé dispersé | |
| Gel N° IV/G-A1₁ exemple 3 contenant 100µg/g Trifarotène micronisé dispersé en présence de 5% de composé lipophile (Triglycérides d'acides caprique/caprylique) réparti dans les microcapsules | |
| Gel N° VI/G-A1₁ exemple 3 contenant 100 µg/g Trifarotène micronisé dispersé en présence de 20% de composé lipophile (Triglycérides d'acides caprique/caprylique) réparti dans les microcapsules | |

| Application | |
|---|---|
| Application | 5mg/cm² |
| Quantité d'actif appliqué | 380-436 ng/cm² |
| Nombre de cellule par formulation | 30 (6 cellules par temps d'exposition) |
| Nombre de donneur par formulation | 3 |
| Temps d'exposition | 1h ; 3h ; 6h ; 8h et 24h |

| Dosage échantillons | |
|---|---|
| Lavage compartiment donneur et essuyage | |
| 1er strip | "Excès" / Dose non absorbée |
| Stratum corneum (2-15 strips max) | |
| Epiderme Derme | Peau Totale |
| Liquide Récepteur | Dose absorbée |
| Analyses LC/MS | |
| Limite de quantification | 0,01 ng/ml |

La formule crème de référence est une crème huile dans eau contenant 0,01% de Trifarotène solubilisé dans la phase grasse interne.

La formule gel de référence est un gel contenant 0,01% Trifarotène dispersé dont la composition de base est identique à celles des gels IV/G-A1₁ et VI/G-A1₁ de l'exemple 3. Le gel de référence est exempt de microcapsules.

| Ingrédients / Noms INCI | Composition (% m/m) |
|---|---|
| Trifarotène micronisé | 0,01 |
| Méthyl parabène | 0,2 |
| Propyl parabène | 0,1 |
| Sodium acryloyldiméthyltaurate copolymère/ isohexadécane/polysorbate 80 | 4 |
| Sodium docusate | 0,05 |
| Disodium edetate | 0,1 |
| Glycérol | 4 |
| Propylène glycol | 4 |
| Poloxamer P124 | 0,2 |
| Acide lactique (qs pH 3,5-4) | Qsp pH |
| Eau purifiée | Qsp 100 |

### Résultats :

Les résultats présentés en Figure 1 montrent la quantité pénétrée en pourcentage de la dose appliquée (% dose appliquée) selon les différents compartiments cutanés après 24h d'exposition. La quantité totale pénétrée correspond à la quantité de principe actif dosé les différents compartiments de la peau (stratum corneum, épiderme, derme) et le liquide récepteur.

Les résultats présentés en Figure 2 montrent le profil de pénétration du Trifarotène avec les différentes formulations testées sur 24 heures.

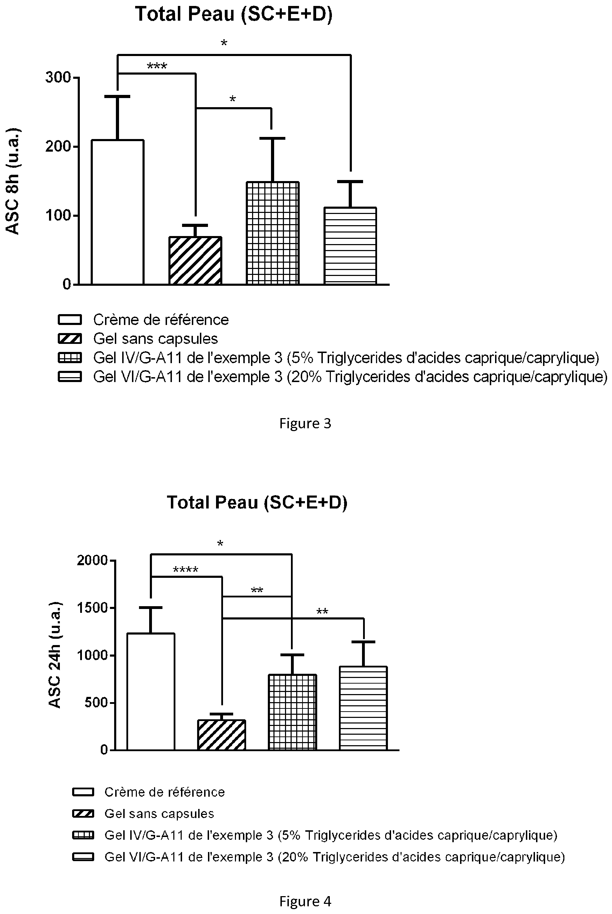

Les Figures 3 et 4 présentent les aires sous la courbes (ASC) calculées respectivement après temps 8 heures correspondant aux temps d'exposition les plus courts et 24 heures correspondant aux temps d'exposition les plus longs. Les SSC ont été calculées pour chaque cinétique (n=6). Elles ont été ensuite statistiquement comparées par un test *one* way ANOVA suivi d'un test de Dunnett

Les valeurs des ASC à 8 heures et à 24 heures sont respectivement les suivantes :

| Valeurs ASC | Crème référence | Gel sans capsules | Gel IV/G-A11 (5% huile) | Gel VI/G-A11 (20% huile) |
|---|---|---|---|---|
| **ASC 8h** | | | | |
| Moyenne | 209,60 | 69,11 | 149,20 | 111,80 |
| Ecart-type | 63,43 | 16,98 | 63,47 | 38,16 |

| **ASC 24h** | | | | |
|---|---|---|---|---|
| Moyenne | 1234 | 318,3 | 797,3 | 886,3 |
| Ecart-type | 272,5 | 63,57 | 211,4 | 259,1 |

### Conclusions :

Selon la Figure 1, quelle que soit la formulation testée, la distribution cutanée du Trifarotène est localisée essentiellement dans le stratum corneum et l'épiderme, et plus modérément dans le derme. En présence de microcapsules, la quantité totale pénétrée du Trifarotène est significativement supérieure au gel de référence dans lequel le Trifarotène est dispersé seul sans présence de microcapsules.
Pour le gel de référence comprenant le Trifarotène dispersé sans microcapsules, la quantité pénétrée totale est de l'ordre de 4.93% à 24h.
Pour la crème de référence comprenant le Trifarotène solubilisé, la quantité pénétrée totale est de l'ordre de 20,83%.
Pour les gels comprenant le Trifarotène dispersé en présence de microcapsules, la quantité pénétrée varie de 13,23 à 19,21%.
Ainsi, les microcapsules permettent à juste titre d'assurer une meilleure pénétration du Trifarotène dispersé, et dans ce cas précis de 2,7 à 4 fois plus en présence de capsules comparé au gel de référence avec l'actif dispersé et ne contenant pas de microcapsules.

En présence de microcapsules contenant 20% de Triglycérides d'acides caprique/caprylique soit le gel VI/G-A1₁ de l'exemple 3, la quantité totale pénétrée de Trifarotène dispersé est comparable à celle obtenue avec la crème de référence dans laquelle le Trifarotène est solubilisé.

D'après la Figure 2, le profil de libération du Trifarotène dispersé est très différent de ceux obtenus avec la crème de référence et les gels contenant des microcapsules. Dans le cas des gels, ce profil est d'autant plus rapide que la quantité d'huile et donc de microcapsules est importante.

D'après la Figure 3 représentant les ASC à 8 heures correspondant aux temps d'exposition les plus courts, le gel ne contenant pas de microcapsules présente une exposition de l'actif dans la peau significativement différente de celle obtenue avec la référence crème et les gels contenant des microcapsules. Par contre, les gels avec microcapsules présentent une exposition cutanée équivalente quelle que soit la teneur en huile : 5 ou 20%. Seul le gel avec 5% d'huile sous forme de microcapsules est significativement différent du gel ne contenant pas de microcapsules.

D'après la Figure 4 représentant les ASC à 24 heures correspondant aux temps d'exposition les plus longs, le gel ne contenant pas de microcapsules présente une exposition de l'actif dans la peau significativement différente de celle obtenue avec la référence crème et les gels contenant des microcapsules. Le gel contenant le plus d'huile sous forme de microcapsules (20%) présente une exposition cutanée équivalente à celle obtenue avec la référence crème dans laquelle l'actif est solubilisé. Plus la quantité de microcapsules est importante et donc la quantité de solvant, plus l'exposition cutanée aux temps longs est importante.

Ainsi sur des temps longs, un gel contenant un actif dispersé en présence de microcapsules contentant un solvant de l'actif permet d'obtenir une exposition cutanée équivalente à celle d'une formulation dans laquelle ce même actif est solubilisé tout en limitant l'exposition cutanée aux temps les plus courts. Ceci permet de présenter un avantage avec des actifs irritants comme par exemple les rétinoïdes et en particulier le Trifarotène.

Cette étude de pénétration cutanée in vitro a démontré que la présence de microcapsules contenant un corps gras solvant de l'actif permet d'obtenir des quantités totales pénétrées de Trifarotène équivalentes à celle d'une formule de référence dans laquelle ce même actif est solubilisé.

De la même manière, cette étude démontre également que la présence de microcapsules contenant un corps gras solvant de l'actif permet également de nettement augmenter la quantité totale pénétrée de Trifarotène dispersé au sein d'une composition de type gel.

### Exemple 7 : Données de solubilité et de stabilité du Composé A2 dans différents corps gras et composés type solvants organiques non volatils

La solubilité et stabilité de l'actif a été évaluée par chromatographie en phase liquide couplée à un détecteur UV (HPLC-UV).

| Nom INCI (nom commercial) | Solubilité maximale ou visuelle * (%m/m) (TA) | Stabilité (3M) (TA/40°C) |
|---|---|---|
| Diméthyl capramide (Spectrasolv DMDA) | 20-30* | NR |
| Diethylène glycol monoéthyl ether (Transcutol HP) | 13,9 | Stable ** |
| Diisopropyl adipate (Schercemol Dia Ester) | <1,0* | NR |
| PPG-15 stéaryl éther (Arlamol PS11E-LQ) | <1,0* | NR |
| Triglycérides d'acides caprique / caprylique / (Miglyol® 812N) | <0,1* | NR |

| | | |
|---|---|---|
| ** apparition d'une coloration à TA et 40°C | | |

Suite aux résultats de cette étude de solubilité, on constate que le diméthyl capramide et le diéthylène glycol monoéthyl éther sont adaptés pour solubiliser le Composé A2.

Suite à ces résultats, le diméthyl capramide et le diéthylène glycol monoéthyl éther sont les solvants préférés choisis pour être insérés au sein du cœur huileux dans les microcapsules en présence de triglycérides d'acides caprique/caprylique .

### Exemple 8: Exemples de compositions de type gel selon l'invention contenant le Composé (A2) dispersé en présence de microcapsules lipidiques réalisées à partir des émulsions primaires de l'exemple 1

Afin de réaliser des compositions de type gel VII/G-A2₁, VII/G-A2₂, V/G-A2₁ et VIII/G-A2₁ selon l'invention, une quantité d'émulsion primaire correspondante préparée selon l'exemple 1 a été prélevée et diluée dans une base gel.

Pour obtenir 100 grammes de gel contenant environ 5% d'huile répartie au sein des microcapsules, 17,784 grammes d'émulsion primaire est ajoutée dans une formulation contenant 1% de Composé A2 micronisé dispersé (cas du gel VII/G-A2₁).

Pour obtenir 100 grammes de gel contenant environ 10% d'huile répartie au sein des microcapsules, 35,855 grammes d'émulsion primaire est ajoutée dans une formulation contenant 1% de Composé A2 micronisé dispersé (cas du gel VII/G-A2₂ et V/G-A2₁).

Pour obtenir 100 grammes de gel contenant environ 20% d'huile répartie au sein de microcapsules, 71,71 grammes d'émulsion primaire est ajoutée dans une formulation contenant 1% de Composé A2 micronisé dispersé (cas du gel VIIIG-A2₁).

Les émulsions primaires VII conduisent respectivement aux compositions gels VII/G-A2₁ et VII/G-A2₂, décrites dans le tableau ci-après.

Les émulsions primaires V et VIII conduisent respectivement aux compositions gels V/G-A2₁ et VIII/G-A2₁, décrites dans le tableau ci-après.

Des exemples de compositions de type gel obtenues selon l'invention sont donc les suivants :

| **Ingrédients** | **Composition (% m/m)** | | | |
|---|---|---|---|---|
| | **VII/G-A2₁** | **VII/G-A2₂** | **V/G-A2₁** | **VIII/G-A2₁** |
| Composé A2 micronisé | 1 | 1 | 1 | 1 |
| Diméthyl capramide | 1 | 2 | -- | -- |
| Diéthylène glycol monoéthyl éther | -- | -- | -- | 8 |
| Triglycérides d'acides caprique / caprylique | 4 | 8 | 10 | 12 |
| Lécithine hydrogénée | 0,72 | 1,4 | 1,4 | 2,9 |
| Sodium acryloyldiméthy ltaurate copolymère/ isohexadécane/p olysorbate 80 | 4 | 4 | 4 | 4 |
| Acide benzoïque | 0,1 | 0,1 | 0,1 | 0,1 |
| Potassium sorbate | 0,1 | 0,1 | 0,1 | 0,1 |
| Disodium edetate | 0,1 | 0,1 | 0,1 | 0,1 |
| Glycérol | 5 | 5 | 5 | 5 |
| Cycloméhicone | 1,5 | 1,5 | 1,5 | 1,5 |
| Acide citrique (qsp pH 5-6) | Qsp pH | Qsp pH | Qsp pH | Qsp pH |
| Eau purifiée | Qsp 100 | Qsp 100 | Qsp 100 | Qsp 100 |

### Exemple 9: Caractérisation des compositions de l'exemple 8 de type gel selon l'invention, selon le composé lipophile mis en œuvre et le pourcentage de mélange lipophile ou de nature lipophile réparti dans les microcapsules

Dans les présents exemples, l'équipement qui a été utilisé pour la réalisation des émulsions primaire est le Magic Lab.
Le mode de dispersion préféré pour la lécithine hydrogénée en présence de triglycérides d'acides caprique/caprylique avec le diméthyl capramide ou de diéthylène glycol monoéthyl éther est 100% dans la phase aqueuse.

La composition gel VII/G-A2₁ contient 1% en composé A2 micronisé dispersé, en présence d'environ 5% d'huile répartie au sein des microcapsules.

| Emulsion primaire / Huile | Caractérisations | Résultats |
|---|---|---|
| **Composition VII/G-A2₁** | | |
| **VII/** diméthyl capramide - Triglycérides d'acides caprique/caprylique | Observation macroscopique | Gel blanc |
| | Observation microscopique | Capsules de taille micrométrique et présence de cristaux de composé A2 |
| | pH | 4.85 |
| | Viscosité RV, S06, 10 rpm | 49 100 cP |

Les compositions gels VII/G-A2₂ et V/G-A2₁ contiennent 1% en composé A2 micronisé dispersé, en présence d'environ 10% de mélange lipophile ou de nature lipophile réparti au sein des microcapsules.

| Emulsion primaire / Huile | Caractérisations | Résultats |
|---|---|---|
| **Composition VII/G-A2₂** | | |
| VII/ diméthyl capramide- | Observation macroscopique | Gel blanc |
| Triglycérides d'acides caprique/caprylique | Observation microscopique | Capsules de taille micrométrique et présence de cristaux de composé A2 |
| | pH | 4.90 |
| | Viscosité RV, S06, 10 rpm | 75 500 cP |

| **Composition V/G-A2₁** | | |
|---|---|---|
| V/ Triglycérides d'acides caprique/caprylique | Observation macroscopique | Gel blanc |
| | Observation microscopique | Capsules de taille micrométrique et présence de cristaux de composé A2 |
| | pH | 5,20 |
| | Viscosité RV, S06, 5 rpm | 135 000 cP |

La composition gel VIII/G-A2₁ contient 1% en composé A2 micronisé dispersé, en présence d'environ 20% de mélange lipophile ou de nature lipophile réparti au sein des microcapsules.

| Emulsion primaire / Huile | Caractérisations | Résultats |
|---|---|---|
| **Composition VIII/G-A2₁** | | |
| VIII/ diéthylène glycol monoéthyl éther - Triglycérides d'acides caprique/caprylique | Observation macroscopique | Gel blanc |
| | Observation microscopique | Capsules de taille micrométrique et présence de cristaux de composé A2 |
| | pH | 4,95 |
| | Viscosité RV, S07, 5 rpm | 230 000 cP |

Ces résultats montrent qu'il est possible de réaliser un gel contenant un principe actif dispersé, à savoir le Composé A2, et contenant des microcapsules avec un mélange lipophile comprenant un composé non huileux de type solvant organique non-volatil (Diéthylène glycol monoéthyl éther).

### Exemple 10 : Etude de stabilité des gels de l'exemple 9 selon le composé lipophile mis en œuvre et selon le pourcentage de mélange lipophile ou de nature lipophile réparti dans les microcapsules.

Gel obtenu à partir de la composition V/G-A2₁ de l'exemple 8 (10% Triglycérides d'acides caprique/caprylique)

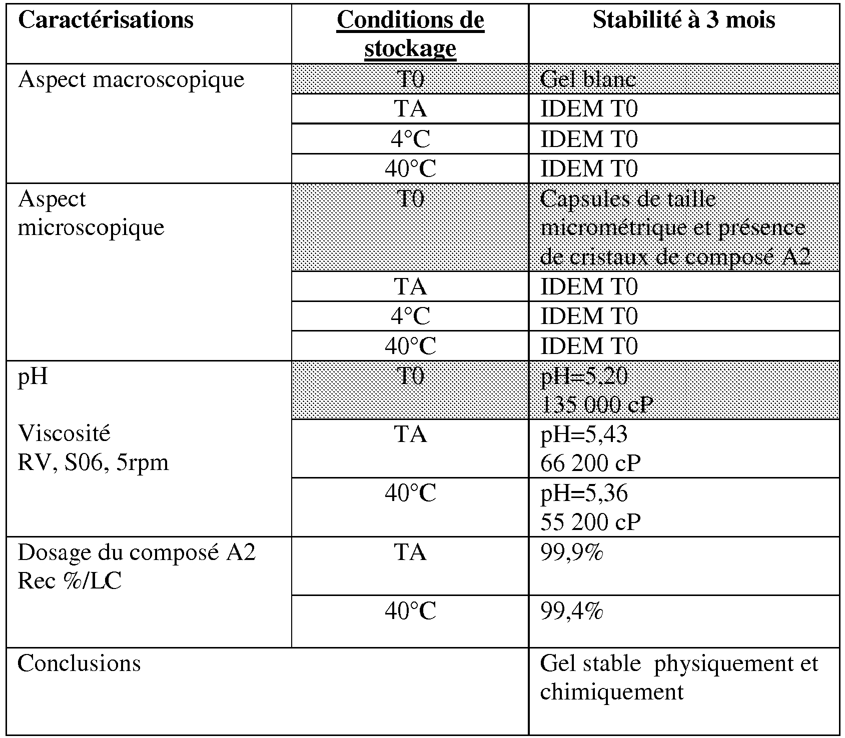

Gel obtenu à partir de la composition VIII/G-A21 de l'exemple 8 (8% diéthylène glycol monoéthyl éther - 12% Triglycérides d'acides caprique/caprylique)

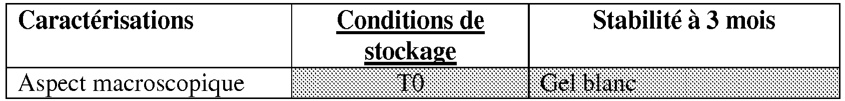

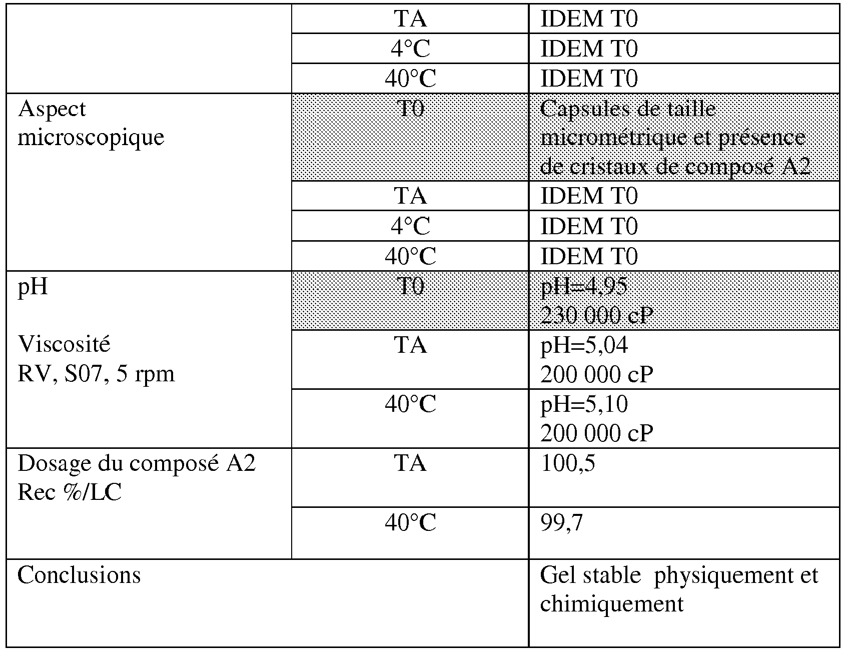

Les résultats montrent que l'on peut obtenir des gels stables physiquement à trois mois à température ambiante et à 40°C en présence d'un principe actif dispersé, à savoir le composé A2.

Le gel VIII/ G-A2₁ est stable physiquement sans problématique d'apparition de coloration, après trois mois à température ambiante et à 40°C, avec dans le cœur huileux du diéthylène glycol monoéthyl éther. Cette absence de coloration n'avait pas été obtenue avec une formulation type gel dans laquelle le Composé A2 était solubilisé à 1%

### Exemple 11: Etude de pénétration cutanée in vitro du composé A2 dispersé dans des formulations gels en présence de microcapsules réalisées selon l'exemple 1

### Conditions de l'étude :

Dans cette étude, les formulations ont été appliquées pendant 24 heures à la surface de la peau. A la fin de l'application, le composé A₂ est quantifié dans les différents compartiments de la peau : stratum corneum, épiderme, derme et liquide récepteur selon une méthode de bioanalyse validée réalisée par spectrométrie de masse en tandem par ionisation électrospray positive, et utilisant un appareil Xevo (Waters). La limite de quantification pour le composé A₂ est de2 ng/mL. Les conditions de LC/MS/MS mises au point ont permis de détecter jusqu'à 0,1% de la dose appliquée dans chacun des compartiments (dose non absorbée, stratum, épiderme, derme et liquide récepteur).

Les détails de l'application cutanée sont donnés dans le tableau ci-dessous.

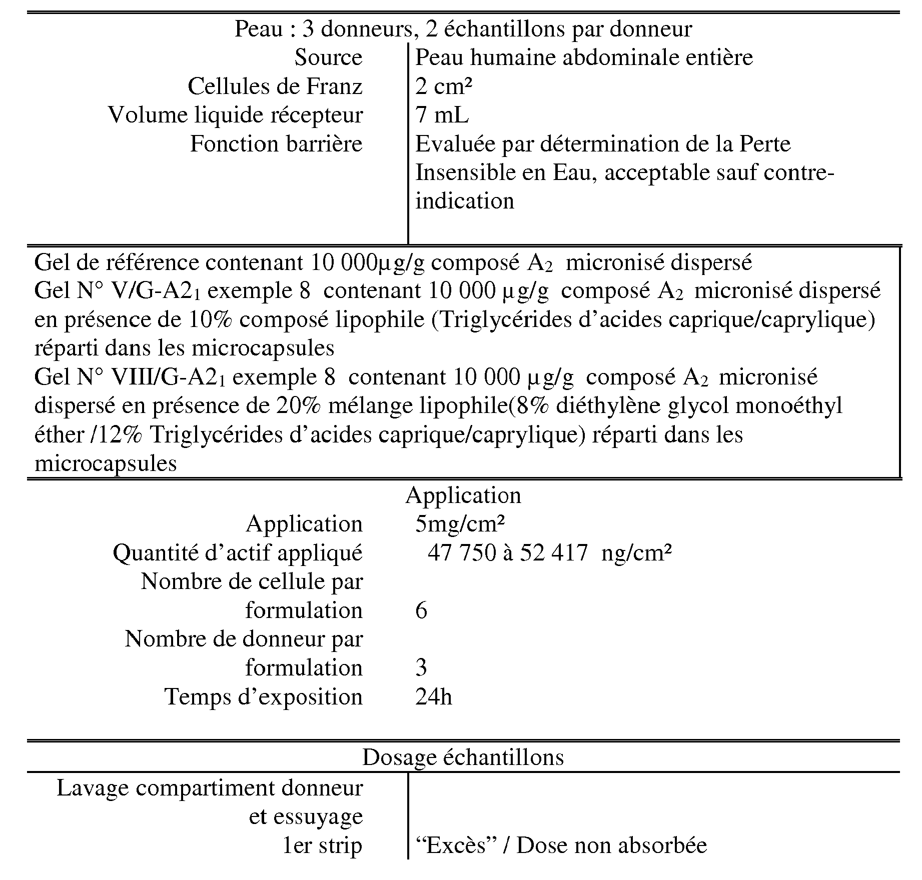

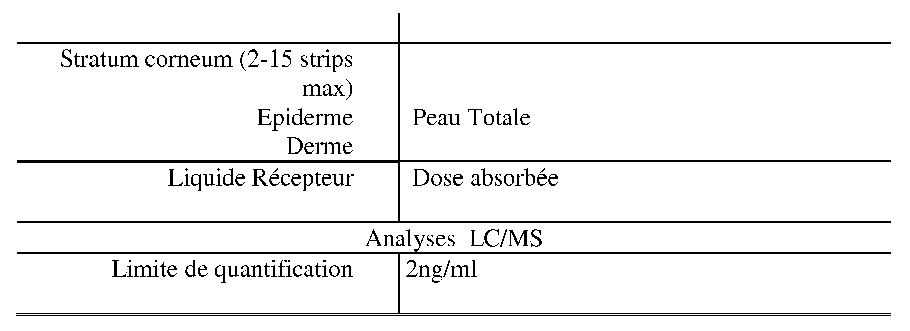

La formule gel de référence est un gel contenant 1% de composé A₂ micronisé dont la composition de base est identique à celles des gels V/G-A2₁ et VIII/G-A2₁ de l'exemple 8. Le gel de référence est exempt de microcapsules.

| Ingrédients / Noms INCI | Composition (% m/m) |
|---|---|
| composé A₂ micronisé | 1,0 |
| Sodium acryloyldiméthyltaurate copolymère/ isohexadécane/polysorbate 80 | 4,0 |
| Acide benzoïque | 0,1 |
| Potassium sorbate | 0,1 |
| Disodium edetate | 0,1 |
| Glycérol | 5,0 |
| Cyclométhicone | 1,5 |
| Acide citrique(qsp pH 5-6) | Qsp pH |
| Eau purifiée | Qsp 100 |

### Résultats :

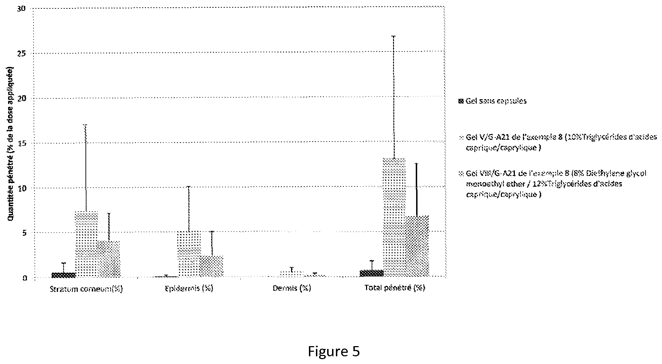

Les résultats présentés en Figure 5 montrent la quantité pénétrée en pourcentage de la dose appliquée (% dose appliquée) selon les différents compartiments cutanés.

### Conclusions :

Quelle soit la formulation testée, la distribution cutanée du composé A₂ dispersé est localisée essentiellement dans le stratum corneum et l'épiderme, et plus modérément dans le derme.

La quantité totale pénétrée du composé A₂ dans les gels contenant le composé A₂ dispersé en présence de microcapsules est supérieure au gel de référence dans lequel le composé A₂ est dispersé seul sans microcapsules.
Avec le gel de référence comprenant le composé A₂ dispersé sans microcapsules, la quantité pénétrée totale est de l'ordre de 0,7%.
Pour les gels comprenant le composé A₂ dispersé en présence de microcapsules, la quantité totale pénétrée varie de 6,72 à 13,15%.
Ainsi, les microcapsules permettent à juste titre d'assurer une meilleure pénétration du composé A₂ dispersé, et dans ce cas précis de 9,6 à 18,8 fois plus en présence de capsules comparé au gel de référence avec l'actif dispersé et ne contenant pas de microcapsules.

L'objet de notre invention, qui annonce l'amélioration de la pénétration de principes actifs dispersés grâce à l'utilisation de microcapsules lipidiques permettant de solubiliser l'actif lors de l'application sur la peau est nettement confirmée dans les conditions de cette étude et avec à titre d'exemple le composé A₂.
Cette étude de pénétration cutanée in vitro a démontré que la présence de microcapsules contenant un mélange lipophile solvant de l'actif permet d'augmenter très significativement la quantité totale pénétrée de Composé A2 dispersé dans un gel par rapport à un même gel exempte de microcapsules.

Il a été aussi mis en évidence dans cette étude que la nature du cœur huileux est un facteur qui influe sur le niveau de pénétration du principe actif.

### Exemple 12 : Données de solubilité du Composé A3 dans différents composés lipophiles ou composés de type solvants organiques non volatils ainsi que dans des mélanges de ces composés

La solubilité de l'actif a été évaluée par chromatographie en phase liquide couplée à un détecteur UV (HPLC-UV).

| Nom INCI (nom commercial) | Solubilité maximale ou visuelle * (%m/m) (TA) |
|---|---|
| Diméthyl sulfoxide (Procipient DMSO) | >20%* |
| Diisopropyl adipate (Schercemol Dia Ester) | 0,02 |
| PPG-15 stéaryl éther (Arlamol PS11E-LQ) | 0,01 |
| C12-15 alkyl lactate (Ceraphyl 41) | 0,04 |
| Triglycérides d'acides caprique / caprylique / (Miglyol® 812N) | < limite de détection |
| 10/90 % Diméthyl sulfoxide/PPG-15 stérayl éther | 0,07 |
| 10/90 % Diméthyl sulfoxide/ Diisopropyl adipate | 0,38 |
| 10/90 % Diméthyl sulfoxide/C12-15 alkyl lactate | 0,12 |
| 20/80 % Diméthyl sulfoxide/Schercemol Dia Ester | 3,15 |
| 20/80 % Diméthyl sulfoxide/C12-15 alkyl lactate | 0,74 |

Suite aux résultats de cette étude de solubilité, le diméthyl sulfoxide est adapté pour solubiliser le Composé A3.

C'est le composé de type organique non volatil préféré et choisi pour être inséré au sein du cœur huileux dans les microcapsules en présence de diisopropyl adipate.

### Exemple 13: Exemples de compositions de type gel selon l'invention contenant le Composé A3 dispersé en présence de microcapsules lipidiques réalisées à partir des émulsions primaires de l'exemple 1.

Afin de réaliser une composition de type gel IX/G-A3₁, selon l'invention, une quantité d'émulsion primaire correspondante préparée selon l'exemple 1 a été prélevée et diluée dans une base gel.

Pour obtenir 100 grammes de gel contenant environ 10% d'huile répartie au sein des microcapsules, 35,855 grammes d'émulsion primaire est ajoutée dans une formulation contenant 3% de Composé A3 micronisé dispersé (cas du gel VII/G-A3₁).

L'émulsion primaire IX conduit respectivement à la composition gel IX/G-A3₁ décrite dans le tableau ci-après.
Un exemple de composition de type gel obtenue selon l'invention est donc la suivante :

| **Ingrédients** | **Composition (%m/m)** |
|---|---|
| | **IX/G-A3₁** |
| Composé A3 micronisé | 3,0 |
| Diméthyl sulfoxide | 2,0 |
| Triglycérides d'acides caprique/caprylique | 8,0 |
| Lécithine hydrogénée | 1,40 |
| Sodium acryloyldiméthyltaurate copolymère/ isohexadécane/polysorbate 80 | 4,0 |
| Méthyl parabène | 0,2 |
| Propyl parabène | 0,1 |
| Butyl hydroxytoluène | 0,036 |
| Eau purifiée | Qsp 100 |

### Exemple 14: Caractérisation de la compositions de l'exemple 13 de type gel selon l'invention.

Dans le présent exemple, l'équipement qui a été utilisé pour la réalisation de l'émulsion primaire est le Magic Lab.
Le mode de dispersion préféré pour la lécithine hydrogénée avec le mélange Diméthyl sulfoxide/Diisopropyl adipate est 100% dans la phase aqueuse.

La composition gel IX/G-A3₁ contient 3% du Composé A3 micronisé dispersé, en présence d'environ 10% d'un mélange lipophile répartie au sein des microcapsules.

| Emulsion primaire / Huile | Caractérisations | Résultats |
|---|---|---|
| **Composition IX/G-A1₁** | | |
| IX/ Diméthyl sulfoxide/ | Observation macroscopique | Gel blanc |
| Triglycérides d'acides caprique/caprylique | Observation microscopique | Capsules de taille micrométrique et présence de cristaux du composé A3 |
| | pH | 5,92 |

Ces résultats montrent qu'il est possible de réaliser un gel contenant un principe actif dispersé, à savoir le Composé A3, et contenant des microcapsules avec un mélange lipophile comprenant un composé non huileux de type solvant organique non volatil (Diméthyl sulfoxide).

## Revendications

1. Composition pharmaceutique comprenant au moins un principe actif et des microcapsules lipidiques de taille micrométrique comprenant une phase interne huileuse, et une enveloppe non polymérique obtenue à partir d'au moins un composé lipidique choisi parmi les lipides amphiphiles, dispersés au sein d'un même véhicule pharmaceutiquement acceptable, et dans laquelle les microcapsules lipidiques sont exemptes dudit principe actif.

2. Composition selon la revendication 1, **caractérisée en ce que** le principe actif dispersé est le Trifarotène, le composé (S)-4-(2,4-dihydroxyphenyl)-N-(1-phenylethyl)piperidine-1-carboxamide, ou le composé (S)-N-hydroxy-3-[4-(2-méthyl-quinolin-4-ylméthoxy)-benzènesulfonylamino]-2-[4-(propane-2-sulfonyl)-pipérazin-1-yl]-propionamide.

3. Composition selon la revendication 1 ou 2, **caractérisée en ce que** les microcapsules lipidiques de taille micrométrique sont dispersées dans une phase aqueuse.

4. Composition selon l'une quelconque des revendications 1 à 3, **caractérisée en ce que** 50% des microcapsules présentent au moins une taille moyenne comprise entre 1 et 80 µm, préférentiellement comprise entre 1 et 50 µm, et plus particulièrement comprise entre 1 et 20 µm.

5. Composition selon l'une quelconque des revendications 1 à 4, **caractérisée en ce que** le composé lipidique constituant l'enveloppe des microcapsules est choisi parmi les lécithines hydrogénée ayant une quantité en poids de phosphatidylcholine supérieure à 85%.

6. Composition selon l'une quelconque des revendications 1 à 4, **caractérisée en ce que** le composé lipidique constituant l'enveloppe des microcapsules est présent en une quantité comprise entre 0,01 et 10%, de préférence entre 0,05 et 5% en poids, et plus préférentiellement entre 0,1 et 1% en poids par rapport au poids total de la composition.

7. Composition selon l'une quelconque des revendications 1 à 6, **caractérisée en ce que** les microcapsules sont exemptes de co-surfactant, de solvant organique volatil ou de polymère.

8. Composition selon l'une des revendications 1 à 7, **caractérisée en ce que** la phase interne huileuse des microcapsules comprend au moins un corps gras liquide ou semi-liquide à température ambiante.

9. Composition selon la revendication 8, **caractérisée en ce que** la phase interne huileuse est composée de triglycérides, d'esters d'acide gras, d'éthers de polyéthylène glycol, ou du diméthyl isosorbide.

10. Composition comprenant 0,001 à 1% en poids d'un actif dispersé, de préférence le Trifarotène, le composé (S)-4-(2,4-dihydroxyphenyl)-N-(1-phenylethyl) piperidine-1-carboxamide, ou le composé (S)-N-hydroxy-3-[4-(2-méthyl-quinolin-4-ylméthoxy)-benzènesulfonylamino] -2- [4- (propane-2-sulfonyl)-pipérazin-1-yl] - propionamide, au sein d'un véhicule pharmaceutiquement acceptable, par rapport au poids total de la composition, et
a) des microcapsules lipidiques composées de :
a. 0,1 à 5% de lécithine hydrogénée avec une teneur en phosphatidylcholine hydrogénée supérieure à 85% ; et
b. 1 à 30% de corps gras huileux et éventuellement non huileux, de préférence des triglycérides, des esters d'acide gras, des éthers de polyéthylène glycol, et du diméthyl isosorbide.

11. Composition selon l'une des revendications 1 à 10, **caractérisée en ce que** le véhicule pharmaceutiquement acceptable est un gel, une crème ou une solution.

12. Composition selon l'une des revendications 1 à 11, **caractérisée en ce qu'**elle se présente sous une forme adaptée pour une administration topique.

13. Composition selon l'une quelconque des revendications 1 à 12, pour son utilisation en tant que médicament.

14. Composition selon l'une quelconque des revendications 1 à 12, pour son utilisation dans le traitement des affections dermatologiques, de préférence l'acné, les ichtyoses, les états ichtyosiformes, l'hyperkératose palmoplantaire et/ou le psoriasis.

15. Procédé de préparation des compositions selon l'une quelconque des revendications 1 à 12 **caractérisé en ce qu'**il comprend les étapes suivantes:
(i) préparation de l'émulsion primaire par :
a) préparation d'une phase huileuse chauffée à 75°C.
b) dispersion du composé lipidique, et plus préférentiellement de la lécithine hydrogénée, dans une phase aqueuse, chauffée à 75°C
c) incorporation de la phase huileuse sur la phase aqueuse déjà présente dans l'appareil sous agitation à une vitesse inférieure à 16 000 t/min, et
d) laisser circuler le mélange jusqu'au retour à température ambiante ; et
(ii) incorporation de l'émulsion primaire dans le véhicule pharmaceutiquement acceptable contenant le principe actif dispersé.

## Patentansprüche

1. Pharmazeutische Zusammensetzung, die mindestens einen Wirkstoff und Lipidmikrokapseln von mikrometrischer Größe enthält, die eine ölige innere Phase und eine nichtpolymere Hülle enthalten, die aus mindestens einer Lipidverbindung erhalten wird, die aus den amphiphilen Lipiden ausgewählt wird, die in einem gleichen pharmazeutisch verträglichen Träger dispergiert sind, und in der die Lipidmikrokapseln frei von dem besagten Wirkstoff sind.

2. Zusammensetzung nach Anspruch 1, **gekennzeichnet dadurch, dass** der dispergierte Wirkstoff Trifaroten, die Verbindung (S)-4-(2,4-Dihydroxyphenyl)-N-(1-Phenylethyl)piperidin-1-Carboxamid oder die Verbindung (S)-N-Hydroxy-3-[4-(2-Methyl-Chinolin-4-ylmethoxy)-Benzolsulfonylamino]-2-[4-(Propan-2-Sulfonyl)-Piperazin-1-yl]-Propionamid ist.

3. Zusammensetzung nach Anspruch 1 oder 2, **gekennzeichnet dadurch, dass** die Lipidmikrokapseln von mikrometrischer Größe in einer wässrigen Phase dispergiert sind.

4. Zusammensetzung nach einem der Ansprüche 1 bis 3, **gekennzeichnet dadurch, dass** 50% der Mikrokapseln mindestens eine Durchschnittsgröße zwischen 1 und 80 µm, vorzugsweise zwischen 1 und 50 µm und insbesondere zwischen 1 und 20 µm aufweisen.

5. Zusammensetzung nach einem der Ansprüche 1 bis 4, **gekennzeichnet dadurch, dass** die Lipidverbindung, die die Hülle der Mikrokapseln bildet, aus den hydrierten Lecithinen mit einer Gewichtsmenge an Phosphatidylcholin von mehr als 85% ausgewählt wird.

6. Zusammensetzung nach einem der Ansprüche 1 bis 4, **gekennzeichnet dadurch, dass** die Lipidverbindung, die die Hülle der Mikrokapseln bildet, in einer Menge zwischen 0,01 und 10 Gew.-%, vorzugsweise zwischen 0,05 und 5 Gew.-% und besonders bevorzugt zwischen 0,1 und 1 Gew.-% im Verhältnis zum Gesamtgewicht der Zusammensetzung vorhanden ist.

7. Zusammensetzung nach einem der Ansprüche 1 bis 6, **gekennzeichnet dadurch, dass** die Mikrokapseln frei von Co-Tensid, flüchtigem organischem Lösungsmittel oder Polymer sind.

8. Zusammensetzung nach einem der Ansprüche 1 bis 7, **gekennzeichnet dadurch, dass** die ölige innere Phase der Mikrokapseln mindestens eine Fettsubstanz enthält, die bei Raumtemperatur flüssig oder halbflüssig ist.

9. Zusammensetzung nach Anspruch 8, **gekennzeichnet dadurch, dass** die ölige innere Phase aus Triglyceriden, Fettsäureestern, Polyethylenglykolethern oder Dimethylisosorbid besteht.

10. Zusammensetzung mit 0,001 bis 1 Gew.-% eines dispergierten Wirkstoffs, vorzugsweise Trifaroten, der Verbindung (S)-4-(2,4-Dihydroxyphenyl)-N-(1-Phenylethyl)piperidine-1-Carboxamid oder der Verbindung (S)-N-Hydroxy-3-[4-(2-Methyl-Chinolin-4-ylmethoxy)-Benzolsulfonylamino]-2-[4-(Propan-2-Sulfonyl)-Piperazin-1-yl]-Propionamid in einem pharmazeutisch verträglichen Träger im Verhältnis zum Gesamtgewicht der Zusammensetzung, und
a) Lipidmikrokapseln, die aus Folgendem bestehen:
a. 0,1 bis 5% hydriertem Lecithin mit einem Gehalt an hydriertem Phosphatidylcholin von mehr als 85%; und
b. 1 bis 30% öliger und gegebenenfalls nicht öliger Fettsubstanz, vorzugsweise Triglyceride, Fettsäureester, Polyethylenglykolether und Dimethylisosorbid.

11. Zusammensetzung nach einem der Ansprüche 1 bis 10, **gekennzeichnet dadurch, dass** der pharmazeutisch verträgliche Träger ein Gel, eine Creme oder eine Lösung ist.

12. Zusammensetzung nach einem der Ansprüche 1 bis 11, **gekennzeichnet dadurch, dass** sie sich in einer Form präsentiert, die zur topischen Verabreichung geeignet ist.

13. Zusammensetzung nach einem der Ansprüche 1 bis 12 zur Verwendung als Medikament.

14. Zusammensetzung nach einem der Ansprüche 1 bis 12 zur Verwendung bei der Behandlung von dermatologischen Zuständen, vorzugsweise Akne, Ichthyose, ichthyosiforme Zustände, palmoplantare Hyperkeratose und/oder Psoriasis.

15. Verfahren zum Herstellen von Zusammensetzungen nach einem der Ansprüche 1 bis 12, **gekennzeichnet dadurch, dass** es die folgenden Schritte umfasst:
(i) Herstellen der Primäremulsion durch:
a) Herstellen einer öligen Phase, die auf 75°C erhitzt wird.
b) Dispergieren der Lipidverbindung und besonders bevorzugt des hydrierten Lecithins in einer wässrigen Phase, die auf 75°C erhitzt wird
c) Inkorporieren der öligen Phase in die bereits vorhandene wässrige Phase in der Vorrichtung unter Rühren bei einer Geschwindigkeit von weniger als 16.000 U/min, und
d) die Mischung zirkulieren lassen, bis sie wieder Raumtemperatur erreicht hat; und
(ii) Inkorporieren der Primäremulsion in den pharmazeutisch verträglichen Träger, der den dispergierten Wirkstoff enthält.

## Claims

1. Pharmaceutical composition comprising at least one active ingredient and micrometer-size lipid microcapsules comprising an oily internal phase, and a nonpolymeric envelope obtained from at least one lipid compound chosen from amphiphilic lipids, dispersed in a same pharmaceutically acceptable vehicle, and wherein the lipid microcapsules are free of said active ingredient.

2. Composition according to claim 1, **characterized in that** the dispersed active ingredient is Trifarotene, the compound (S)-4-(2,4-dihydroxyphenyl)-N-(1-phenylethyl)piperidine-1-carboxamide, or the compound (S)-N-hydroxy-3-[4-(2-methyl-quinolin-4-ylmethoxy)-benzenesulfonylamino]-2-[4-(propane-2-sulfonyl)-piperazin-1-yl]-propionamide.

3. Composition according to claim 1 or 2, **characterized in that** the micrometer-size lipid microcapsules are dispersed in an aqueous phase.

4. Composition according to any of claims 1 to 3, **characterized in that** 50% of the microcapsules have at least an average size comprised between 1 and 80 µm, preferably comprised between 1 and 50 µm, and more particularly comprised between 1 and 20 µm.

5. Composition according to any of claims 1 to 4, **characterized in that** the lipid compound constituting the envelope of the microcapsules is chosen from hydrogenated lecithins having a phosphatidylcholine quantity by weight of more than 85%.

6. Composition according to any of claims 1 to 4, **characterized in that** the lipid compound constituting the envelope of the microcapsules is present in a quantity of between 0.01 and 10%, preferably between 0.05 and 5% by weight, and more preferably between 0.1 and 1% by weight relative to the total weight of the composition.

7. Composition according to any of claims 1 to 6, **characterized in that** the microcapsules are free of co-surfactant, volatile organic solvent or polymer.

8. Composition according to one of claims 1 to 7, **characterized in that** the oily internal phase of the microcapsules comprises at least one fatty substance which is liquid or semi-liquid at room temperature.

9. Composition according to claim 8, **characterized in that** the oily internal phase is composed of triglycerides, fatty acid esters, polyethylene glycol ethers, or dimethyl isosorbide.

10. Composition comprising 0.001 to 1% by weight of a dispersed active agent, preferably Trifarotene, the compound (S)-4-(2,4-dihydroxyphenyl)-N-(1-phenylethyl)piperidine-1-carboxamide, or the compound (S)-N-hydroxy-3-[4-(2-methyl-quinolin-4-ylmethoxy)-benzenesulfonylamino]-2-[4-(propane-2-sulfonyl)-piperazin-1-yl]-propionamide, in a pharmaceutically acceptable vehicle, relative to the total weight of the composition, and
a) lipid microcapsules composed of
a. 0.1 to 5% hydrogenated lecithin with a hydrogenated phosphatidylcholine content of more than 85%; and
b. 1 to 30% oily and optionally non-oily fatty substance, preferably triglycerides, fatty acid esters, polyethylene glycol ethers, and dimethyl isosorbide.

11. Composition according to one of claims 1 to 10, **characterized in that** the pharmaceutically acceptable vehicle is a gel, a cream or a solution.

12. Composition according to one of claims 1 to 11, **characterized in that** it is in a form suitable for topical administration.

13. Composition according to any of claims 1 to 12, for use as a medicament.

14. Composition according to any of claims 1 to 12, for use in the treatment of dermatological conditions, preferably acne, ichthyosis, ichthyosiform conditions, palmoplantar hyperkeratosis and/or psoriasis.

15. Process for preparing compositions according to any of claims 1 to 12, **characterized in that** it comprises the following steps:
(i) preparation of the primary emulsion by:
a) preparing an oily phase heated to 75 °C.
b) dispersing the lipid compound, and more preferably hydrogenated lecithin, in an aqueous phase, heated to 75 ° C.
c) adding the oily phase to the aqueous phase that is already present in the apparatus under stirring at a speed of less than 16,000 rpm, and
d) allowing the mixture to circulate until it returns to room temperature; and
(ii) adding the primary emulsion to the pharmaceutically acceptable vehicle containing the dispersed active ingredient.
